# EUROPEAN PATENT APPLICATION

(11) **EP 4 317 183 A1**
(43) Date of publication of application: **07.02.2024**
(21) Application number: 22780805.2
(22) Date of filing: 28.03.2022
(51) Int. Cl.: C07K 16/12, G01N 33/53, G01N 33/543, G01N 33/569

(54) **NEISSERIA GONORRHOEAE DETECTION KIT AND NEISSERIA GONORRHOEAE DETECTION METHOD**

(30) Priority: 29.03.2021 JP 2021056132
(71) Applicant: Asahi Kasei Kabushiki Kaisha, Tokyo 1000006 (JP)
(72) Inventor: ODA, Kotaro, Tokyo 100-0006 (JP); SUGATA, Mika, Tokyo 100-0006 (JP)
(74) Representative: Forstmeyer, Dietmar
(86) International application number: PCT/JP2022/015166
(87) International publication number: WO 2022/210594

(57) **Abstract**

Provided is a kit for detecting Neisseria gonorrhoeae in a sample, as a novel Neisseria gonorrhoeae detection means that has superior Neisseria gonorrhoeae detection sensitivity and can detect Neisseria gonorrhoeae in situ from a variety of samples, such as urine or intraoral fluid. This kit includes a capture antibody that is for capturing a Neisseria gonorrhoeae-originating antigen in a sample, and a detection antibody that has a detection marker and is for marking the Neisseria gonorrhoeae-originating antigen in the sample. The Neisseria gonorrhoeae-originating antigen is a Neisseria gonorrhoeae ribosomal protein L7/L12. One of the capture antibody and the detection antibody is a first monoclonal antibody or a fragment or derivative thereof that produces an antigen-antibody reaction with an epitope including one or more amino acid residues selected from the second to 14th amino acid residues from the N-terminus in the Neisseria gonorrhoeae L7/L12 amino acid sequence indicated in SEQ ID NO: 1. The other of the capture antibody and the detection antibody is a second monoclonal antibody or a fragment or derivative thereof that produces an antigen-antibody reaction with an epitope including one or more amino acid residues selected from the 102nd to 123rd amino acid residues from the N-terminus in the Neisseria gonorrhoeae L7/L12 amino acid sequence indicated in SEQ ID NO: 1.

## Description

### TECHNICAL FIELD

The present invention relates to a kit and a method for detecting Neisseria gonorrhoeae in a sample based on an antigen-antibody reaction.

### BACKGROUND ART

Urethritis, a sexually transmitted disease, is a worldwide problem whose prevention, diagnosis, and treatment are considered important by the World Health Organization (WHO), because the number of patients is increasing worldwide so that there are currently about 78 million patients worldwide. Due to the nature of urethritis, which has a low rate of patients returning to treatment, there is an extremely high need for appropriate antimicrobial prescribing based on *on site* (at the clinic) diagnosis.

There are several species of bacteria that cause urethritis, the most commonly found among which is Neisseria gonorrhoeae. Known methods for detecting Neisseria gonorrhoeae include hybridization using DNA probes (Patent Literature 1: JP-H10-500310 A) and amplification of polynucleotides targeting 16S ribosomal RNA (Patent Literature 2: JP2013-188181 A) are known. However, these methods require a long time for testing and cannot be used as an *on site* (at the clinic) diagnostic tool.

Antibodies that can detect Neisseria gonorrhoeae based on an antigen-antibody reaction are known to use ribosomal protein L7/L12 of Neisseria gonorrhoeae as an antigen (see, e.g., Patent Literature 3: WO2000/006603 A). Other known kits that can detect Neisseria gonorrhoeae based on antigen-antibody reaction are immunoassay kits such as immunochromatography kits (Non-Patent Literature 1: package inserts of Abbott Clearview^{™} Gonorea). However, conventional test kits for detecting Neisseria gonorrhoeae using such known antibodies have a low detection sensitivity (limit of detection (LoD) of approximately 5e6 cfu/mL), making it difficult to detect Neisseria gonorrhoeae in a urine sample. Accordingly, in order to detect Neisseria gonorrhoeae using such conventional kits, a swab must be inserted and rubbed into the urethra to collect an intraurethral sample that contains a higher concentration of Neisseria gonorrhoeae. Since this procedure for collecting samples is extremely burdensome for patients (especially male patients), the use of these test kits has been low, presenting an obstacle to achieving *on site* diagnosis of urethritis and appropriate initial antimicrobial prescriptions based on the *on site* diagnosis results.

According to a previous report (Non-Patent Literature 2: Isbey et al., Genitourinary Medicine, (1997), Vol.73, No.5, pp.378-382), the concentration of Neisseria gonorrhoeae in urine of male patients with urethritis is approximately in the order of 10⁴ to 10⁷ cfu/mL. If a method that can detect Neisseria gonorrhoeae in urine with a concentration in the order of 10⁴ cfu/mL, preferably with a concentration of approximately 10⁴ cfu/mL, can be realized, urine can be used as a sample, thus eliminating the need for the sample collection procedure by inserting and rubbing a swab into the urethra. It is also possible to detect Neisseria gonorrhoeae using other samples such as oral samples (e.g., buccal wipes and gargle effluents) and ocular samples (e.g., conjunctival scraping samples and eye discharges) in which Neisseria gonorrhoeae may be present at low concentrations. This will be of tremendous public health value, because it will allow testing of male urethritis patients at a very small patient burden.

In addition, Neisseria meningitidis, a bacterium of the same genus Neisseria as Neisseria gonorrhoeae, may be detected in urethral secretions as well as Neisseria gonorrhoeae, and has a different drug resistance profile than Neisseria gonorrhoeae. Accordingly, it is desirable to provide means for detecting Neisseria gonorrhoeae that can distinguish Neisseria gonorrhoeae from Neisseria meningitidis.

Furthermore, urine and oral fluid are known to have a wide pH range of approximately 4.5 to 8 and 4 to 7, respectively. Accordingly, in order to detect Neisseria gonorrhoeae using various samples such as urine and oral fluid (e.g., buccal wipes), it is desirable to provide a method that is stable and testable under a wide range of pH conditions.

### LIST OF CITATIONS

### Non-Patent Literature

[Non-Patent Literature 1] Package inserts of Abbott Clearview^{™} Gonorea
[Non-Patent Literature 2] Isbey et al., Genitourinary Medicine, (1997), Vol.73, No.5, pp.378-382

### Patent Literature

[Patent Literature 1] JP-H10-500310 A
[Patent Literature 2] JP2013-188181 A
[Patent Literature 3] WO2000/006603 A

### SUMMARY OF INVENTION

### PROBLEM TO BE SOLVED BY THE INVENTION

The present invention has been made to address the problem mentioned above. An objective of the present invention is to provide a novel method for detecting Neisseria gonorrhoeae on site with excellent sensitivity from various samples such as urine, oral fluid, and ocular samples. Preferably, an objective of the present invention is to provide a novel method for detecting Neisseria gonorrhoeae on site stably under a wide range of pH conditions.

### MEANS TO SOLVE THE PROBLEM

As a result of intensive study, the present inventors have focused on ribosomal protein L7/L12 of Neisseria gonorrhoeae as a Neisseria gonorrhoeae-derived antigen, and have arrived at the concepts of combining a first monoclonal antibody which causes an antigen-antibody reaction with an epitope containing one or more amino acid residues selected from the 2nd to 14th amino acid residues of Neisseria gonorrhoeae L7/L12 (first epitope) and a second monoclonal antibody which causes an antigen-antibody reaction with an epitope containing one or more amino acid residues selected from the 102nd to 123rd amino acid residues of Neisseria gonorrhoeae L7/L12 (second epitope), using one of the first and second monoclonal antibodies as a capture antibody and the other as a detection antibody, and detecting Neisseria gonorrhoeae based on an immune reaction between the Neisseria gonorrhoeae-derived antigen in the sample, the capture antibody, and the detection antibody to form a structure sandwiching the Neisseria gonorrhoeae-derived antigen, whereby it becomes possible to detect Neisseria gonorrhoeae at much lower concentrations than conventional methods, and also to detect Neisseria gonorrhoeae on site in various samples such as urine, oral fluid, and ocular samples, thereby completing the present invention.

Specifically, some aspects of the present invention include the following.
[Aspect 1] A kit for detecting Neisseria gonorrhoeae in a sample, comprising:
   a capture antibody for capturing a Neisseria gonorrhoeae-derived antigen in the sample; and
   a detection antibody having a detection label for labeling the Neisseria gonorrhoeae-derived antigen in the sample,
   wherein Neisseria gonorrhoeae is detected via an immune reaction between the Neisseria gonorrhoeae-derived antigen in the sample, the capture antibody, and the detection antibody to form a structure sandwiching the Neisseria gonorrhoeae-derived antigen,
   wherein the Neisseria gonorrhoeae-derived antigen is a ribosomal protein L7/L12 of Neisseria gonorrhoeae,
   wherein one of the capture antibody and the detection antibody is a first monoclonal antibody or its fragment or a derivative thereof, which causes an antigen-antibody reaction with an epitope containing one or more amino acid residues selected from the 2nd to 14th amino acid residues from the N-terminal of the amino acid sequence of Neisseria gonorrhoeae L7/L12 shown in SEQ ID NO:1,
   wherein the other of the capture antibody and the detection antibody is a second monoclonal antibody or its fragment or a derivative thereof, which causes an antigen-antibody reaction with an epitope containing one or more amino acid residues selected from the 102nd to 123rd amino acid residues from the N-terminal of the amino acid sequence of Neisseria gonorrhoeae L7/L12 shown in SEQ ID NO:1.
[Aspect 2] The kit according to Aspect 1, wherein the second monoclonal antibody comprises:
   as the amino acid sequence of a heavy chain variable region, an amino acid sequence having a homology of 80% or more to the amino acid sequence of SEQ ID NO:7, and,
   as the amino acid sequence of a light chain variable region, an amino acid sequence having a homology of 80% or more to the amino acid sequence of SEQ ID NO:8.
[Aspect 3] The kit according to Aspect 1 or 2, wherein the first monoclonal antibody comprises:
   as the amino acid sequence of a heavy chain variable region, an amino acid sequence having a homology of 80% or more to the amino acid sequence of SEQ ID NO:9, and
   as the amino acid sequence of a light chain variable region, an amino acid sequence having a homology of 80% or more to the amino acid sequence of SEQ ID NO: 10; or
   as the amino acid sequence of a heavy chain variable region, an amino acid sequence having a homology of 80% or more to the amino acid sequence of SEQ ID NO:11, and
   as the amino acid sequence of a light chain variable region, an amino acid sequence having a homology of 80% or more to the amino acid sequence of SEQ ID NO: 12.
[Aspect 4] The kit according to any one of Aspects 1 to 3, wherein the capture antibody is the first monoclonal antibody and the detection antibody is the second monoclonal antibody.
[Aspect 5] The kit according to any one of Aspects 1 to 3, wherein the detection antibody is the first monoclonal antibody and the capture antibody is the second monoclonal antibody.
[Aspect 6] A kit for detecting Neisseria gonorrhoeae in a sample, comprising:
   a capture antibody for capturing a Neisseria gonorrhoeae-derived antigen in the sample; and
   detection antibody having a detection label for labeling the Neisseria gonorrhoeae-derived antigen in the sample,
   wherein Neisseria gonorrhoeae is detected via an immune reaction between the capture antibody, and the detection antibody to form a structure sandwiching the Neisseria gonorrhoeae-derived antigen,
   wherein the Neisseria gonorrhoeae-derived antigen is a ribosomal protein L7/L12 of Neisseria gonorrhoeae, and
   wherein the capture antibody and the detection antibody are selected from:
      (1) a combination where the capture antibody is (a) or (b) below, and the labeling antibody is (c) below; or
      (2) a combination where the labeling antibody is (a) or (b) below, and the capture antibody is (c) below.
         (a) An antibody comprising:
            as the amino acid sequence of a heavy chain variable region, an amino acid sequence having a homology of 80% or more to the amino acid sequence of SEQ ID NO:11, and,
            as the amino acid sequence of a light chain variable region, an amino acid sequence having a homology of 80% or more to the amino acid sequence of SEQ ID NO: 12.
         (b) An antibody comprising:
            as the amino acid sequence of a heavy chain variable region, an amino acid sequence having a homology of 80% or more to the amino acid sequence of SEQ ID NO:9, and,
            as the amino acid sequence of a light chain variable region, an amino acid sequence having a homology of 80% or more to the amino acid sequence of SEQ ID NO: 10.
         (c) An antibody comprising:
            as the amino acid sequence of a heavy chain variable region, an amino acid sequence having a homology of 80% or more to the amino acid sequence of SEQ ID NO:7, and,
            as the amino acid sequence of a light chain variable region, an amino acid sequence having a homology of 80% or more to the amino acid sequence of SEQ ID NO:8.
[Aspect 7] The kit according to any one of Aspects 1 to 6, wherein an efficiency in detecting the Neisseria gonorrhoeae-derived antigen more than 10 times higher than the efficiency in detecting a Neisseria meningitidis -derived antigen.
[Aspect 8] The kit according to any one of Aspects 1 to 6, which does not cause a cross-reaction with bacteria of the genus Mycoplasma, the genus Escherichia, the genus Chlamydia, the genus Pseudomonas, the genus Staphylococcus, the genus Legionella, the genus Streptococcus in the sample.
[Aspect 9] The kit according to any one of Aspects 1 to 8, wherein the sample is derived from oral fluid or urine.
[Aspect 10] The kit according to Aspect 9, wherein the sample is derived from urine of a mammal.
[Aspect 11] The kit according to any one of Aspects 1 to 10, further comprising a carrier for developing the sample and contacting the sample with the detection antibody,
   wherein the carrier has a detection region to which the capture antibody is immobilized.
[Aspect 12] The kit according to Aspect 11, wherein the kit is an immunochromatography kit,
   wherein the kit further comprises a conjugate pad to which the detection antibody is attached.
[Aspect 13] A method for detecting Neisseria gonorrhoeae in a sample using a kit according to any one of Aspects 1 to 12, comprising the steps of:
   (I) capturing and labeling a Neisseria gonorrhoeae-derived antigen in the sample based on an antigen-antibody reaction between the Neisseria gonorrhoeae-derived antigen in the sample, the capture antibody, and the detection antibody; and
   (II) detecting the Neisseria gonorrhoeae-derived antigen in the sample by using the detection label.
[Aspect 14] The method according to Aspect 13, wherein step (I) comprises the steps of:
   (Ia-1) making the sample into contact with the detection antibody to thereby label the Neisseria gonorrhoeae-derived antigen in the sample based on an antigen-antibody reaction between the detection antibody and the Neisseria gonorrhoeae-derived antigen in the sample; and
   (Ia-2) making the sample containing the Neisseria gonorrhoeae-derived antigen labeled with the detection antibody into contact with the capture antibody to thereby capture the Neisseria gonorrhoeae-derived antigen in the sample based on an antigen-antibody reaction between the capture antibody and a complex of the Neisseria gonorrhoeae-derived antigen and the detection antibody.
[Aspect 15] The method according to Aspect 13, wherein step (I) comprises the steps of:
   (Ib-1) making the sample into contact with the capture antibody to thereby capture the Neisseria gonorrhoeae-derived antigen in the sample based on an antigen-antibody reaction between the capture antibody and the Neisseria gonorrhoeae-derived antigen in the sample; and
   (Ib-2) making the sample containing the Neisseria gonorrhoeae-derived antigen captured by the capture antibody into contact with the detection antibody to thereby label the Neisseria gonorrhoeae-derived antigen in the sample based on an antigen-antibody reaction between the detection antibody and a complex of the Neisseria gonorrhoeae-derived antigen and the capture antibody.
[Aspect 16] The method according to any one of Aspects 13 to 15, further comprising the step of, before making the detection antibody and/or the capture antibody into contact with the sample, lysing the bacteria in the sample using a lysing agent,
   wherein the lysing agent is one or more selected from the group consisting of non-ionic surfactant, zwitterionic surfactant, anionic surfactant, lysozyme, lysostaphin, pepsin, glucosidase, galactosidase, achromopeptidase, and β-N-acetyl glucosaminase.
[Aspect 17] The method according to any one of Aspects 13 to 16, whose detection limit of Neisseria gonorrhoeae in a sample is 5e4 cfu/mL or less.
[Aspect 18] A method for producing a kit according to Aspect 11, comprising the step of immobilizing the capture antibody to a detection region of a carrier.
[Aspect 19] A method for producing a kit according to Aspect 12, comprising the steps of:
   immobilizing the capture antibody to a detection region of a carrier;
   attaching the detection antibody to a conjugate pad; and
   arranging the conjugate pad at a position upstream of the detection region of the carrier.
[Aspect 20] The method according to Aspect 18 or 19, further comprising the step of adding the detection label to one of the first and second monoclonal antibodies to thereby prepare the detection antibody,
   wherein the other of the first and second monoclonal antibodies is immobilized to the detection region of the carrier as the capture antibody.
[Aspect 21] The method according to Aspect 20, wherein the first monoclonal antibody is the first monoclonal antibody according to Aspect 1,
   wherein the method further comprises the step of obtaining the first monoclonal antibody by inoculating an animal with an epitope polypeptide having an epitope containing one or more amino acid residues selected from the 2nd to 14th amino acid residues from the N-terminal of the amino acid sequence of Neisseria gonorrhoeae L7/L12 shown in SEQ ID NO:1, obtaining an antiserum containing an antibody which causes an antigen-antibody reaction with the epitope polypeptide, and purifying and separating the antibody from the antiserum.
[Aspect 22] The method according to Aspect 20 or 21, wherein the second monoclonal antibody is the second monoclonal antibody according to Aspect 1,
   wherein the method further comprises the step of obtaining the second monoclonal antibody by inoculating an animal with an epitope polypeptide having an epitope containing one or more amino acid residues selected from the 102nd to 123rd amino acid residues from the N-terminal of the amino acid sequence of Neisseria gonorrhoeae L7/L12 shown in SEQ ID NO:1, obtaining an antiserum containing an antibody which causes an antigen-antibody reaction with the epitope polypeptide, and purifying and separating the antibody from the antiserum.

### EFFECT OF THE INVENTION

The Neisseria gonorrhoeae detection kit and Neisseria gonorrhoeae detection method according to the present invention enable detection of Neisseria gonorrhoeae based on an antigen-antibody reaction at lower concentrations than conventional methods. This makes it possible to detect Neisseria gonorrhoeae on site from a variety of samples such as urine, oral fluid, and ocular samples. In addition, according to a preferable embodiment, Neisseria gonorrhoeae can be detected on site stably under a wide range of pH conditions.

### BRIEF EXPLANATION OF FIGURES

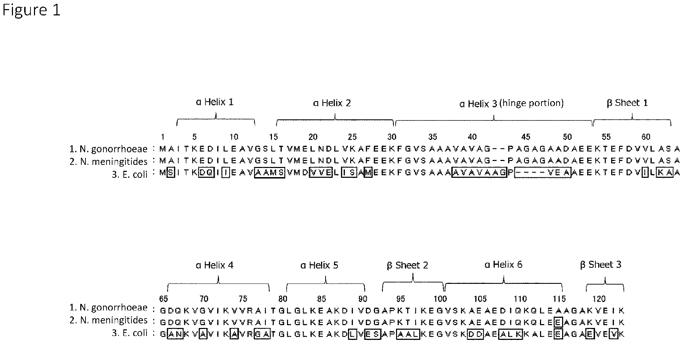

[Figure 1] Figure 1 shows the alignment of the amino acid sequences of ribosomal proteins L7/L12 of Neisseria gonorrhoeae, Neisseria meningitidis, and E. coli. For the L7/L12 amino acid sequence of Neisseria gonorrhoeae, the predicted α-helices and β-sheet forming regions are also shown. For the L7/L12 amino acid sequences of Neisseria meningitidis and E. coli, amino acid residues that differ from those of the L7/L12 amino acid sequence of Neisseria gonorrhoeae are shown with enclosing lines.

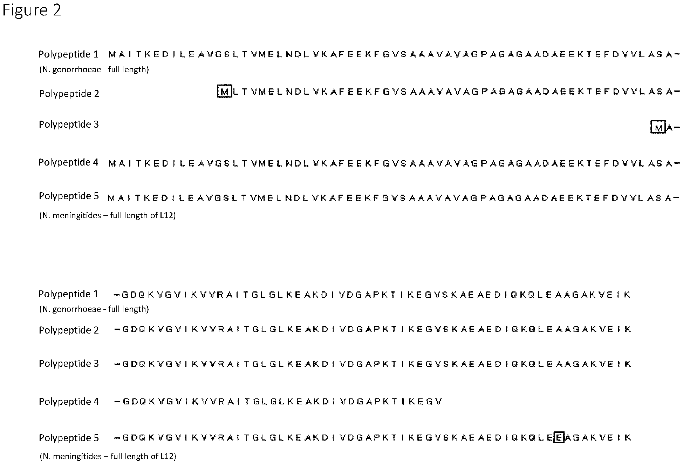

[Figure 2] Figure 2 shows the alignment of the amino acid sequence of ribosomal proteins L7/L12 of Neisseria gonorrhoeae (polypeptide 1), the amino acid sequences of polypeptides 2 to 4, and the amino acid sequence of ribosomal proteins L7/L12 of Neisseria meningitidis (polypeptide 5). For the L7/L12 amino acid sequences of polypeptide 2 to 5, amino acid residues that differ from those of the L7/L12 amino acid sequence of Neisseria gonorrhoeae are shown with enclosing lines.

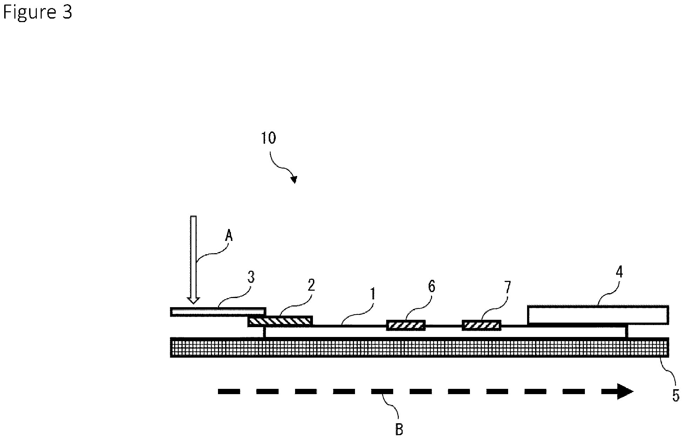

[Figure 3] Figure 3 is a cross-sectional view of a schematic diagram of a strip-shaped detection mechanism, an example of a detection mechanism of a lateral flow type immunochromatographic detection system.

### DESCRIPTION OF EMBODIMENTS

The present invention will be described in detail by referring to the specific embodiments below. However, the present invention should not be limited to the following embodiments, but can be implemented in any form to the extent that it does not depart from the gist of the present invention.

### [I. Introduction]

The term "Neisseria gonorrhoeae" herein refers to a Gram-negative diplococcus belonging to the genus Neisseria. Infection with Neisseria gonorrhoeae is known to cause diseases such as gonococcal urethritis (gonorrhea), keratoconjunctivitis, and pharyngitis in humans. The most common treatment for diseases caused by Neisseria gonorrhoeae infection is the administration of antibiotics, but many bacteria are drug-resistant, and treatment is difficult when the situation worsens. Therefore, there is a need for a simple and immediate means of detecting Neisseria gonorrhoeae infection.

The present invention provides a novel method for on site detection of Neisseria gonorrhoeae from various samples such as urine, oral samples (e.g., buccal wipes and gargle effluents) and ocular samples (e.g., conjunctival scraping samples and eye discharges) with excellent detection sensitivity, and preferably, stable on site detection of Neisseria gonorrhoeae under a wide range of pH conditions. In order to provide such a novel means to detect Neisseria gonorrhoeae, the present inventors focused on the ribosomal protein L7/L12 of Neisseria gonorrhoeae as a Neisseria gonorrhoeae-derived antigen to produce a monoclonal antibody capable of detecting Neisseria gonorrhoeae. The term "ribosomal protein L7/L12," or simply "L7/L12," herein refers to a type of ribosomal protein essential for bacterial protein synthesis and commonly possessed by various bacteria.

Figure 1 shows the alignment of the amino acid sequences of ribosomal proteins L7/L12 of Neisseria gonorrhoeae, Neisseria meningitidis, and E. coli. In addition, the amino acid sequence of the L7/L12 polypeptide of Neisseria gonorrhoeae is shown in SEQ ID NO:1, the amino acid sequence of the L7/L12 polypeptide of Neisseria meningitidis is shown in SEQ ID NO:2, and the amino acid sequence of the L7/L12 polypeptide of E. coli is shown in SEQ ID NO:3s.

The ribosomal protein L7/L12 of Neisseria gonorrhoeae has a dimeric structure consisting of two copies of monomeric molecules each composed of 123 amino acid residues (also referred to as "L7/L12 polypeptide") linked together. As shown in Figure 1, the L7/L12 polypeptide of Neisseria gonorrhoeae includes a conformation (NTD: N-Terminal Domain) formed with the amino acid residues at positions 1 to 40 and another conformation (CTD: C-Terminal Domain) formed with the amino acid residues at positions 58 to 123, which conformations are interconnected via a linker consisting of 17 amino acid residues, which do not form a specific conformation. In addition, two copies of monomeric molecules of L7/L12 polypeptide having such a steric structure associate with each other NTDs to form a dimeric structure.

The amino acid sequence of the L7/L12 polypeptide of Neisseria meningitidis, a bacterium of the same genus Neisseria as Neisseria gonorrhoeae, is very similar to that of the L7/L12 polypeptide of Neisseria gonorrhoeae as shown in Figure 1, differing only in that the amino acid residue at position 115 from the N-terminal is glutamic acid in Neisseria meningitidis, while it is alanine in Neisseria gonorrhoeae. This similarity makes it extremely difficult to identify the L7/L12 polypeptides of Neisseria gonorrhoeae and Neisseria meningitidis based on an antigen-antibody reaction.

On the other hand, the amino acid sequence of the L7/L12 polypeptide of Escherichia coli, a bacterium of the genus Escherichia, which is different from Neisseria gonorrhoeae and Neisseria meningitidis, is significantly different from that of Neisseria gonorrhoeae and Neisseria meningitidis, as shown in Figure 1.

The present inventors have produced monoclonal antibodies capable of binding to various sites of the L7/L12 polypeptide of Neisseria gonorrhoeae, and tested various combinations of these antibodies. As a result, the present inventors have found that a novel Neisseria gonorrhoeae detection method that has excellent detection sensitivity and can detect Neisseria gonorrhoeae on site from various samples such as urine, oral samples (e.g., buccal wipes and gargle effluents) and ocular samples (e.g., conjunctival scraping samples and eye discharges) can be established by combining a first monoclonal antibody which causes an antigen-antibody reaction with an epitope containing one or more amino acid residues selected from the 2nd to 14th amino acid residues of Neisseria gonorrhoeae L7/L12 (hereinafter also referred to as "first epitope") and a second monoclonal antibody which causes an antigen-antibody reaction with an epitope containing one or more amino acid residues selected from the 102nd to 123rd amino acid residues of Neisseria gonorrhoeae L7/L12 (hereinafter also referred to as "second epitope").

Thus, an aspect of the present invention relates to the combination of first and second monoclonal antibodies that causes an antibody-antigen reaction with first and second epitopes, respectively, of Neisseria gonorrhoeae L7/L12 (hereinafter also referred to as "the first Neisseria gonorrhoeae detection antibody of the present invention" and "the second Neisseria gonorrhoeae detection antibody of the present invention," respectively, or simply as "the first antibody of the present invention" and "the second antibody of the present invention," respectively). Another aspect of the present invention relates to a kit for detecting Neisseria gonorrhoeae in a sample (hereinafter also referred to as "the Neisseria gonorrhoeae detection kit of the present invention") and a method for detecting Neisseria gonorrhoeae in a sample (hereinafter also referred to as "the Neisseria gonorrhoeae detection method of the present invention") using the combination of the first and second antibodies of the present invention. These aspects will be described below in turn.

### [II. First and second antibodies for detecting Neisseria gonorrhoeae]

The first and second Neisseria gonorrhoeae detection antibodies of the present invention are monoclonal antibodies or their fragments or derivatives thereof that can cause an antigen-antibody reaction with epitopes containing specific amino acid residues from the ribosomal protein L7/L12 of Neisseria gonorrhoeae.

The term "antibody" used herein refers to a protein that recognizes and binds to a specific antigen or substance, which may also be referred to as an immunoglobulin (Ig). Common antibodies typically have two light chains (light chains) and two heavy chains (heavy chains) that are interconnected by disulfide bonds. There are two classes of light chains, called λ and κ chains, and five classes of heavy chains, called γ, µ, α, δ, and ε chains. Depending on the class of their heavy chains, antibodies are classified into five isotypes: IgG, IgM, IgA, IgD and IgE, respectively.

Heavy chains each include a heavy chain constant (CH) region and a heavy chain variable (VH) region. Light chains each include a light chain constant (CL) region and a light chain variable (VL) region. The light chain constant (CL) region consists of a single domain. The heavy chain constant (CL) region consists of three domains, namely CH1, CH2, and CH3. The light chain variable (VL) region and the heavy chain variable (VH) region each consist of four highly conserved regions called framework regions (FRs; FR-1, FR-2, FR-3, and FR-4) and three hypervariable regions called complementarity-determining regions (CDRs; CDR-1, CDR-2, and CDR-3). The heavy chain constant (CH) region consists of three CDRs (CDR-H1, CDR-H2, and CDR-H3) and four FRs (FR-H1, FR-H2, FR-H3, and FR-H4), which are arranged from the amino terminus to the carboxy terminus in the order of FR-H1, CDR-H1, FR-H2, CDR-H2, FR-H3, CDR-H3, and FR-H4. The light chain constant (CL) region has three CDRs (CDR-L1, CDR-L2, CDR-L3) and four FRs (FR-L1, FR-L2, FR L3, and FR-L4), which are arranged from the amino terminus to the carboxy terminus in the order of FR-L1, CDR-L1, FR-L2, CDR-L2, FR-L3, CDR-L3, and FR-L4. The variable regions of the heavy and light chains contain binding domains that interact with the antigen.

There are two types of antibodies: polyclonal and monoclonal. A polyclonal antibody is usually prepared from the serum of an animal immunized with an antigen and is a mixture of various antibody molecules with different structures. A monoclonal antibody, on the other hand, is an antibody composed of a single type of molecules containing a combination of light chain variable (VL) and heavy chain variable (VH) regions having determined amino acid sequences. The first and second antibodies of the present invention are both monoclonal antibodies. Monoclonal antibodies can be produced from clones derived from antibody-producing cells, or they can be produced using genetic engineering technique, by obtaining nucleic acid molecules having gene sequences encoding amino acids of antibody proteins. It is also a well-known technique to those skilled in the art to improve the binding and specificity of antibodies using genetic information of their heavy chains and light chains or their variable regions and CDRs.

The first and second antibodies of the present invention may be a fragment and/or derivative of an antibody. Fragments of antibodies include F(ab')₂, Fab, Fv, etc. Antibody derivatives include antibodies in which amino acid mutations have been artificially introduced into the constant region(s) of the light and/or heavy chains, antibodies in which the domain configuration of the constant region(s) of the light and/or heavy chains has been modified, antibodies with two or more Fc regions per molecule, glycosylated antibodies, bispecific antibodies, antibody conjugates in which an antibody or antibody fragment is bound to a protein other than the antibody, antibody enzymes, antibody conjugates in which an antibody or antibody fragment is bound to a protein other than an antibody, etc. Antibody conjugates, antibody enzymes, tandem scFv, bispecific tandem scFv, diabody, etc. Furthermore, when the aforementioned antibodies or their fragments or derivatives are derived from non-human animals, chimeric or humanized antibodies in which some or all of the sequences other than the CDRs thereof are replaced with the corresponding sequences of human antibodies are also included in the scope of first and second antibodies of the present invention. When the simple term "antibodies" is used herein, it is intended to also encompass fragments and/or derivatives of antibodies, unless otherwise specified.

The first and second antibodies of the present invention are monoclonal antibodies that produce antigen-antibody reactions with the first and second epitopes, respectively, which contain specific amino acid residues of the L7/L12 amino acid sequence of Neisseria gonorrhoeae shown in SEQ ID NO:1 as described below.

The term "antigen-antibody reaction" herein refers to a reaction in which an antibody recognizes and binds to any component of its antigen. The term "epitope" herein refers to a part of the antigen recognized by the antibody. The length of an epitope is not restricted, but may be within the range of typically 3 amino acid residues or more, particularly 5 amino acid residues or more, or 7 amino acid residues or more, and typically 50 amino acid residues or less, particularly 30 amino acid residues or less, or 20 amino acid residues or less.

The first epitope, with which the first antibody of the present invention causes an antigen-antibody reaction, is an epitope containing one or more amino acid residues selected from the 2nd to 14th amino acid residues from the N-terminal of the amino acid sequence of Neisseria gonorrhoeae L7/L12 shown in SEQ ID NO:1.

The second epitope, with which the second antibody of the present invention causes an antigen-antibody reaction, is an epitope containing one or more amino acid residues selected from the 102nd to 123rd amino acid residues from the N-terminal of the amino acid sequence of Neisseria gonorrhoeae L7/L12 shown in SEQ ID NO:1. Among these, the second epitope may preferably contain at least the 115th amino acid residue from the N-terminus of SEQ ID NO:1, which is the only difference between the L7/L12 sequence of Neisseria gonorrhoeae and that of Neisseria meningitidis.

The first and/or second antibody of the present invention may preferably not cause any cross-reaction with any ingredients derived from bacteria (preferably ribosomal protein L7/L12) other than Neisseria gonorrhoeae or with any other ingredients in the sample. Specifically, the first and/or second antibody of the present invention may preferably not cause any cross-reaction with bacteria of one or more selected from the genus Mycoplasma, the genus Escherichia, the genus Chlamydia, the genus Salmonella, the genus Pseudomonas, the genus Staphylococcus, the genus Legionella, the genus Haemophilus, the genus Bordetella, the genus Moraxella, and the genus Streptococcus. More specifically, the first and/or second antibody of the present invention may preferably not cause any cross-reaction with bacteria of 2 or more, or 3 or more, or 4 or more, or 5 or more, or 6 or more, especially all, of the genera mentioned above.

Among these, the first and/or second antibody of the present invention may preferably not cause any cross-reaction with any ingredients derived from bacteria (preferably ribosomal protein L7/L12) belonging to the same genus Neisseria as Neisseria gonorrhoea. Examples of bacteria belonging to the genus Neisseria include Neisseria meningitidis, Neisseria lactamica, Neisseria sicca, Neisseria cinerea, and Neisseria flavescens.

In particular, the second antibody of the present invention may preferably not cause any cross-reaction with any ingredients (preferably ribosomal protein L7/L12) derived from Neisseria meningitidis. Neisseria meningitidis may be detected in urethral secretions like Neisseria gonorrhoeae, and have a different anti-microbial resistance profile than that of Neisseria gonorrhoeae. Therefore, when detecting Neisseria gonorrhoeae, it may be preferable to also distinguish Neisseria gonorrhoeae from Neisseria meningitidis. In addition, as explained above with reference to Figure 1, the amino acid sequence of the L7/L12 polypeptide of Neisseria meningitidis is extremely similar to that of Neisseria gonorrhoeae, with the only difference in that the 115th amino acid residue from the N-terminal is alanine in Neisseria gonorrhoeae, while it is glutamic acid in Neisseria meningitidis. Therefore, it is extremely difficult to distinguish the L7/L12 polypeptide of Neisseria gonorrhoeae from that of Neisseria meningitidis using conventional methods based on an antigen-antibody reaction. However, the second antibody of the present invention, in its preferred embodiment, can recognize a second epitope containing at least the 115th amino acid residue from the N-terminus of SEQ ID NO:1, which is the only difference between the L7/L12 polypeptides of Neisseria gonorrhoeae and Neisseria meningitidis, and can cause an antigen-antibody reaction therewith. Therefore, the second antibody of the present invention is highly desirable because it can significantly suppress the reactivity to the L7/L12 of Neisseria meningitidis in contrast to the reactivity to the L7/L12 of Neisseria gonorrhoeae, thus making it possible to detect Neisseria gonorrhoeae in a sample with differentiating it from Neisseria meningitidis.

A person skilled in the art can choose to measure an antigen-antibody reaction between an antibody and an epitope/antigen or other components in a solid-phase or liquid-phase system as appropriate. Examples of such methods include, although are not limited to: enzyme-linked immunosorbent assay (ELISA), enzyme immunoassay (EIA), surface plasmon resonance (SPR), fluorescence resonance energy transfer (FRET), and luminescence resonance energy transfer (LRET). In measuring such antigen-antibody binding, the antibody and/or the antigen can be labeled with an enzyme, fluorescent substance, luminescent substance, radioisotope, etc., and the antigen-antibody reaction can be detected using a measurement method suitable for the physical and/or chemical properties of the labeled substance.

The amino acid sequences of the first and second Neisseria gonorrhoeae detection antibodies of the present invention are not limited so long as they can cause antigen-antibody reactions with the first and second epitopes, respectively. However, antibodies having the following amino acid sequences as the respective variable region sequences of the heavy and light chains may be preferred for their high sensitivity.

The heavy chain variable region may preferably have an amino acid sequence with a homology (preferably identity) of 80% or more, particularly 85% or more, or 90% or more, or 95% or more, or 96% or more, or 97% or more, or 99% or more, especially 100%, to at least one amino acid sequence selected from SEQ ID NO:7 (the heavy chain variable region sequence of Antibody NG1 in the Examples), SEQ ID NO:9 (the heavy chain variable region sequence of Antibody NG2 in the Examples), and SEQ ID NO:11 (the heavy chain variable region sequence of Antibody NG3 in the Examples). Among these, the heavy chain variable region may more preferably have an amino acid sequence selected from SEQ ID NO:7, SEQ ID NO:9, and SEQ ID NO:11.

The light chain variable region may preferably have an amino acid sequence with a homology (preferably identity) of 80% or more, particularly 85% or more, or 90% or more, or 95% or more, or 96% or more, or 97% or more, or 99% or more, especially 100%, to at least one amino acid sequence selected from SEQ ID NO:8 (the light chain variable region sequence of Antibody NG1 in the Examples), SEQ ID NO:10 (the light chain variable region sequence of Antibody NG2 in the Examples), and SEQ ID NO:12 (the light chain variable region sequence of Antibody NG3 in the Examples). Among these, the light chain variable region may more preferably have an amino acid sequence selected from SEQ ID NO:8, SEQ ID NO:10, and SEQ ID NO:12.

As used herein, the term "homology" between two amino acid sequences refers to the ratio in which identical or similar amino acid residues appear in each corresponding position when these amino acid sequences are aligned, and the term "identity" between two amino acid sequences refers to the ratio of similar amino acid residues appearing in each corresponding position when these amino acid sequences are aligned. The "homology" and "identity" between two amino acid sequences can be determined using, for example, the BLAST (Basic Local Alignment Search Tool) program (Altschul et al., J. Mol. Biol., (1990), 215(3):403-10). (1990, 215(3):403-10).

Methods for identifying the sequence of each CDR from the respective variable sequences of the heavy and light chains of an antibody include, for example, the Kabat method (Kabat et al., The Journal of Immunology, 1991, Vol. 147, No. 5, pp. 1709-1719) and the Chothia method (Al Lazikani et al., Journal of Molecular Biology, 1997, Vol. 273, No. 4, pp. 927-948). These methods are common knowledge in this field, but it is possible to refer to, for example, the website of Dr. Andrew C.R. Martin's Group (http://www.bioinf.org.uk/abs/).

Similar amino acids include, for example, amino acids that are classified under the same group in the following classification based on the polarity, charge, and size of each amino acid (in any case, each amino acid type is indicated by a single-letter code).
*Aromatic amino acids: F, H, W, Y;
* Aliphatic amino acids: I, L, V;
*Hydrophobic amino acids: A, C, F, H, I, K, L, M, T, V, W, Y;
*Charged amino acids: D, E, H, K, R, etc.;
*Positively charged amino acids: H, K, R;
*Negatively charged amino acids: D, E;
*Polar amino acids: C, D, E, H, K, N, Q, R, S, T, W, Y;
*Small amino acids: A, C, D, G, N, P, S, T, V, etc.; and
*Micro amino acids: A, C, G, S.

Similar amino acids also include, for example, amino acids that are classified under the same group in the following classification based on the side chain of each amino acid (in any case, each amino acid type is indicated by a single-letter code).
*Amino acids having an aliphatic side chain: G, A, V, L, I;
*Amino acids having an aromatic side chain: F, Y, W;
*Amino acids having a sulfur-containing side chain: C, M;
*Amino acids having an aliphatic hydroxyl side chain: S, T;
*Amino acids having a basic side chain: K, R, H;
*Amino acids having an acidic side chain and their amide derivatives: D, E, N, Q.

The specific antibody by combining the sequences of the heavy chain and light chain variable regions mentioned above are not restricted, but examples thereof include the following.
*An antibody having, as the heavy chain variable region sequence, an amino acid sequence having a homology of 80% or more to the amino acid sequence of SEQ ID NO:11 and, as the light chain variable region sequence, an amino acid sequence having a homology of 80% or more to the amino acid sequence of SEQ ID NO:12.
*An antibody having, as the heavy chain variable region sequence, an amino acid sequence having a homology of 80% or more to the amino acid sequence of SEQ ID NO:9 and, as the light chain variable region sequence, an amino acid sequence having a homology of 80% or more to the amino acid sequence of SEQ ID NO:10.
*An antibody having, as the heavy chain variable region sequence, an amino acid sequence having a homology of 80% or more to the amino acid sequence of SEQ ID NO:7 and, as the light chain variable region sequence, an amino acid sequence having a homology of 80% or more to the amino acid sequence of SEQ ID NO:8.

Among these, preferable antibodies as the first monoclonal antibody include: an antibody having, as the heavy chain variable region sequence, an amino acid sequence having a homology of 80% or more to the amino acid sequence of SEQ ID NO:11 and, as the light chain variable region sequence, an amino acid sequence having a homology of 80% or more to the amino acid sequence of SEQ ID NO:12; and an antibody having, as the heavy chain variable region sequence, an amino acid sequence having a homology of 80% or more to the amino acid sequence of SEQ ID NO:9 and, as the light chain variable region sequence, an amino acid sequence having a homology of 80% or more to the amino acid sequence of SEQ ID NO:10. Preferable antibodies as the second monoclonal antibody including an antibody having, as the heavy chain variable region sequence, an amino acid sequence having a homology of 80% or more to the amino acid sequence of SEQ ID NO:7 and, as the light chain variable region sequence, an amino acid sequence having a homology of 80% or more to the amino acid sequence of SEQ ID NO:8.

When these exemplary first and second monoclonal antibodies are used in the Neisseria gonorrhoeae detection method and the Neisseria gonorrhoeae detection kit of the present invention, the combination of the capture antibody and the labeling antibody may be, although is not limited to, either the combination (1) or (2) below.
(1) The combination of the antibody of (a) or (b) below as the capture antibody and the antibody of (c) below as the labeling antibody.
(2) The combination of the antibody of (a) or (b) below as the labeling antibody and the antibody of (c) below as the capture antibody.
   (a) An antibody having, as the heavy chain variable region sequence, an amino acid sequence having a homology of 80% or more to the amino acid sequence of SEQ ID NO:11 and, as the light chain variable region sequence, an amino acid sequence having a homology of 80% or more to the amino acid sequence of SEQ ID NO:12.
   (b) An antibody having, as the heavy chain variable region sequence, an amino acid sequence having a homology of 80% or more to the amino acid sequence of SEQ ID NO:9 and, as the light chain variable region sequence, an amino acid sequence having a homology of 80% or more to the amino acid sequence of SEQ ID NO:10.
   (c) An antibody having, as the heavy chain variable region sequence, an amino acid sequence having a homology of 80% or more to the amino acid sequence of SEQ ID NO:7 and, as the light chain variable region sequence, an amino acid sequence having a homology of 80% or more to the amino acid sequence of SEQ ID NO:8.

Methods for producing the first and second antibodies of the invention, including the antibodies exemplified above, may include, although are not particularly limited to, the following methods.

The first step is to prepare a polypeptide having an amino acid sequence of all or part of L7/L12 of Neisseria gonorrhoeae to be detected, or an amino acid sequence having a homology (preferably identity) of 90% or more, especially 95% or more, or 96% or more, or 97% or more, or 99% or more, especially 100% therewith (hereinafter referred to as the "epitope polypeptide"). The specific amino acid sequence of the epitope polypeptide is not restricted. However, for producing the first antibody of the present invention, it may be preferred to use a polypeptide having an amino acid sequence corresponding to the first epitope (an epitope containing one or more amino acid residues selected from the 2nd to 14th amino acid residues of SEQ ID NO:1) mentioned above, or an amino acid sequence having a homology (preferably identity) of 90% or more, especially 95% or more, or 96% or more, or 97% or more, or 99% or more, especially 100% therewith (hereinafter referred to as "first epitope polypeptide"). On the other hand, for producing the first antibody of the present invention, it may be preferred to use a polypeptide having an amino acid sequence corresponding to the second epitope (an epitope containing one or more amino acid residues selected from the 102nd to 123rd amino acid residues of SEQ ID NO:1) mentioned above, or an amino acid sequence having a homology (preferably identity) of 90% or more, especially 95% or more, or 96% or more, or 97% or more, or 99% or more, especially 100% therewith (hereinafter referred to as "second epitope polypeptide").

The thus-prepared epitope polypeptide is then inoculated into an animal, optionally together with adjuvants as necessary, and the serum is collected to obtain an antiserum containing antibodies (polyclonal antibodies including candidates for the first and/or second antibody of the present invention) that cause antigen-antibody reactions with the epitope polypeptide mentioned above. Examples of animals to be inoculated include sheep, horses, goats, rabbits, mice, rats, etc., of which sheep and rabbits are especially preferred for polyclonal antibody production. The antibodies may be purified and fractionated from the obtained antiserum and screened for antigen-antibody reactivity with the first and/or second epitope, and for no cross-reactivity with other specific components (e.g., Neisseria meningitidis L7/L12), using known methods to obtain the desired the first and/or second antibody of the present invention. Furthermore, monoclonal antibodies can be obtained by isolating antibody-producing cells that produce desired antibody molecules and fusing them with myeloma cells to produce hybridomas capable of autonomous growth.

Once the desired antibodies are obtained by the above procedure, the structure of each antibody, specifically part or all of the amino acid sequences of the heavy chain constant (CH) region, heavy chain variable (VH) region, light chain constant (CL) region, and/or light chain variable (VL) region, can be analyzed using known amino acid sequence analysis methods. It is also known to those skilled in the art to modify the amino acid sequences of the desired antibodies thus obtained to improve their binding ability and specificity. Furthermore, it is also possible to design other antibodies likely to have similar antigen specificity by using all or part of the amino acid sequences of each desired antibody (especially the amino acid sequences of all or part of the heavy chain variable (VH) region and the light chain variable (VL) region, especially the amino acid sequence of each CDR) and, if necessary, by combining them with part of amino acid sequences of known antibodies (especially the amino acid sequences of each FR of the heavy chain variable (VH) and light chain variable (VL) regions, and optionally of the heavy chain variable (VH) and light chain variable (VL) regions).

If the amino acid sequence of a part (CDR or variable region) of a desired antibody is known, a nucleic acid molecule having base sequences encoding all or part of the amino acid sequences of the desired antibody can be produced by a known method, and the antibody can be produced by genetic engineering using such a nucleic acid molecule. Furthermore, it is also possible to create a vector or plasmid for expressing each component of the desired antibody from such a nucleic acid sequence, and introduce it into a host cell (e.g., a mammalian cell, insect cell, plant cell, yeast cell, or microbial cell) to produce the antibody. In addition, modifications can be made to the structures of the constant regions of the antibody or to its sugar chains in order to improve the efficiency of the obtained antibody or to avoid its side effects, using techniques well known to those skilled in the art.

Those explained above, i.e., methods for producing the first and/or second antibody of the present invention, nucleic acid molecules encoding the antibody of the present invention, vectors or plasmids containing such nucleic acid molecules, cells containing such nucleic acid molecules, vectors or plasmids, hybridomas producing the first and/or second antibody of the present invention, etc., are also included in the scope of the present invention.

The techniques for making and modifying the antibodies described herein are all known to those skilled in the art, and may be carried out by referring to, e.g., Antibodies; A laboratory manual, E. Harlow et al. Likewise, the molecular biological techniques described herein (e.g., amino acid sequencing methods, methods for designing and producing nucleic acid molecules, methods for designing and producing vectors and plasmids, etc.) are also all known to those skilled in the art, and may be carried out by referring to, e.g., Molecular Cloning, A laboratory manual, Cold Spring Harbor Laboratory Press, Shambrook, NY. Harbor Laboratory Press, Shambrook, J. et al.

### [III. Neisseria gonorrhoeae detection method]

The Neisseria gonorrhoeae detection method of the present invention includes the steps of:
(I) capturing and labeling a Neisseria gonorrhoeae-derived antigen in the sample based on an antigen-antibody reaction between the Neisseria gonorrhoeae-derived antigen in the sample, the capture antibody, and the detection antibody; and
(II) detecting the Neisseria gonorrhoeae-derived antigen in the sample by using the detection label.

In this method, the Neisseria gonorrhoeae-derived antigen is a ribosomal protein L7/L12 of Neisseria gonorrhoeae, while one of the capture antibody and the detection antibody is the first antibody of the present invention and the other is the second antibody of the present invention.

Either the capture antibody or the detection antibody may be the first antibody of the present invention, and either may be the second antibody of the present invention. When the capture antibody is the first antibody of the present invention and the detection antibody is the second antibody of the present invention, the capture antibody causes an antigen-antibody reaction with the first epitope of Neisseria gonorrhoeae L7/L12 to capture it, while the detection antibody causes an antigen-antibody reaction with the second epitope of Neisseria gonorrhoeae L7/L12 to label it for detection. On the other hand, when the capture antibody is the second antibody of the present invention and the detection antibody is the first antibody of the present invention, the capture antibody causes an antigen-antibody reaction with the second epitope of Neisseria gonorrhoeae L7/L12 to capture it, detection antibody causes an antigen-antibody reaction with the first epitope of Neisseria gonorrhoeae L7/L12 to label it for detection.

Specifically, the combination of the capture antibody and the labeling antibody used in the Neisseria gonorrhoeae detection method of the present invention may be, although is not limited to, either the combination (1) or (2) below.
(1) The combination of the antibody of (a) or (b) below as the capture antibody and the antibody of (c) below as the labeling antibody.
(2) The combination of the antibody of (a) or (b) below as the labeling antibody and the antibody of (c) below as the capture antibody.
   (a) An antibody having, as the heavy chain variable region sequence, an amino acid sequence having a homology of 80% or more to the amino acid sequence of SEQ ID NO:11 and, as the light chain variable region sequence, an amino acid sequence having a homology of 80% or more to the amino acid sequence of SEQ ID NO:12.
   (b) An antibody having, as the heavy chain variable region sequence, an amino acid sequence having a homology of 80% or more to the amino acid sequence of SEQ ID NO:9 and, as the light chain variable region sequence, an amino acid sequence having a homology of 80% or more to the amino acid sequence of SEQ ID NO:10.
   (c) An antibody having, as the heavy chain variable region sequence, an amino acid sequence having a homology of 80% or more to the amino acid sequence of SEQ ID NO:7 and, as the light chain variable region sequence, an amino acid sequence having a homology of 80% or more to the amino acid sequence of SEQ ID NO:8.

Among them, from the viewpoint of achieving applicability to a wide range of pH conditions as described below, the combination of the capture antibody and labeling antibody may preferably be, although is not limited to, either the combination (1) or (2) below.
(1) The combination of the antibody of (a) below as the capture antibody and the antibody of (c) below as the labeling antibody.
(2) The combination of the antibody of (a) below as the labeling antibody and the antibody of (c) below as the capture antibody.
   (a) An antibody having, as the heavy chain variable region sequence, an amino acid sequence having a homology of 80% or more to the amino acid sequence of SEQ ID NO:11 and, as the light chain variable region sequence, an amino acid sequence having a homology of 80% or more to the amino acid sequence of SEQ ID NO:12.
   (c) An antibody having, as the heavy chain variable region sequence, an amino acid sequence having a homology of 80% or more to the amino acid sequence of SEQ ID NO:7 and, as the light chain variable region sequence, an amino acid sequence having a homology of 80% or more to the amino acid sequence of SEQ ID NO:8.

According to Embodiment A of the Neisseria gonorrhoeae detection method of the present invention, the step (I) may include the steps of:
(Ia-1) making the sample into contact with the detection antibody to thereby label the Neisseria gonorrhoeae-derived antigen in the sample based on an antigen-antibody reaction between the detection antibody and the Neisseria gonorrhoeae-derived antigen; and
(Ia-2) making the sample containing the Neisseria gonorrhoeae-derived antigen labeled with the detection antibody into contact with the capture antibody, an antigen-antibody reaction between the capture antibody and a complex of the Neisseria gonorrhoeae-derived antigen and the detection antibody to thereby capture the Neisseria gonorrhoeae-derived antigen in the sample.

According to Embodiment B of the Neisseria gonorrhoeae detection method of the present invention, the step (I) may include the steps of:
(Ib-1) making the sample into contact with the capture antibody to thereby capture the Neisseria gonorrhoeae-derived antigen in the sample based on an antigen-antibody reaction between the capture antibody and the Neisseria gonorrhoeae-derived antigen; and
(Ib-2) making the sample containing the Neisseria gonorrhoeae-derived antigen captured by the capture antibody into contact with the detection antibody, an antigen-antibody reaction between the detection antibody and a complex of the Neisseria gonorrhoeae-derived antigen and the capture antibody to thereby label the Neisseria gonorrhoeae-derived antigen in the sample.

In the Neisseria gonorrhoeae detection method of the present invention, which of the two embodiments, Embodiment A and Embodiment B, may be determined according to the type of immunoassay method and the type of samples actually used. Examples of immunoassay methods include, although are not limited to, various known immunological assays such as ELISA using antibody-loaded microtiter plates; latex particle agglutination assay using latex particles (e.g. polystyrene latex particles) loaded with antibodies; immunochromatography using antibody-loaded membranes; and sandwich assay using a detection antibody labeled with colored or chromogenic particles, enzymes or fluorophores, etc., and a capture antibody immobilized on a solid-phase carrier such as magnetic particles, etc.

The following explanation will be made for cases where the Neisseria gonorrhoeae detection method of the present invention in accordance with Embodiment A is carried out using the immunochromatography method as the immunoassay method. However, each of the conditions can be modified as appropriate when using other immunoassay methods.

The types of the solid-phase carrier used for the capture antibody are not restricted, but specific examples include: porous membranes made of cellulose, nitrocellulose, cellulose acetate, nylon, PVDF (polyvinylidene difluoride), glass fiber, etc.; flow channels made of glass, plastic, PDMS (poly(dimethylsiloxane)), silicone, etc.; thread, paper, fiber, etc.

The method for attaching an antibody to the solid-phase carrier is also not restricted, but specific examples include fixation via physisorption using the hydrophobicity of the antibody and fixation via chemical bonding using a functional group of the antibody.

The type of detection label used for the detection antibody is not restricted and may be selected as appropriate in accordance with the detection method. Specific examples include: metal colloids such as gold colloids, platinum colloids, and palladium colloids; non-metal colloids such as selenium colloids, alumina colloids, and silica colloids; insoluble granular materials such as colored resin particles, dye colloids, colored liposomes, etc.; enzymes that catalyze chromogenic reactions such as alkaline phosphatase, peroxidase, luciferase, etc.; fluorescent dyes, radioisotopes; and chemiluminescent labels, bioluminescent labels, electrochemiluminescent labels, etc.

The method for attaching the label to the antibody is also not restricted, but specific examples of methods that can be used include physical adsorption using the hydrophobicity of the antibody, chemisorption using a functional group of the antibody, etc.

The subject of detection by the Neisseria gonorrhoeae test method of the present invention is not limited and may be any subject in which Neisseria gonorrhoeae infection is sought to be detected, but may typically be mammals, preferably humans.

The samples to be used for Neisseria gonorrhoeae detection are also not restricted, and may be any biological samples derived from subjects so long as they may contain Neisseria gonorrhoeae as a cause for urethritis. Examples of samples include: urine of the subject; intra-urethral samples, which can be obtained by inserting and rubbing a swab into the subject's urethra; oral samples (oral liquids such as buccal wipes and gargle effluents); ocular samples (e.g., conjunctival abrasion samples and eye discharges); vaginal abrasion samples; and cervical abrasion samples. An advantage of the Neisseria gonorrhoeae test method of the present invention is that urine, which is less burdensome for the subject to obtain, can be used as the sample. Specifically, as mentioned above, because conventional Neisseria gonorrhoeae test kits using immunochromatography (Non-Patent Literature 1) have a low detection sensitivity (limit of detection (LoD) of approximately 5e6 cfu/mL), It was necessary to obtain intra-urethral samples with high gonococcal concentrations, which was a significant burden for the subjects (especially male subjects). On the other hand, the Neisseria gonorrhoeae test method of the present invention may preferably have a very high sensitivity with a detection limit for Neisseria gonorrhoeae in a sample of 5e4 cfu/mL or less, more preferably 3e4 cfu/mL or less, still more preferably 2e4cfu/mL or less. This allows for the subject's urine to be used as the sample, which greatly reduces the burden on the subject (especially male subjects).

The first and second antibodies of the present invention to be used in the Neisseria gonorrhoeae test method of the present invention produce an antigen-antibody reaction by recognizing specific epitopes present in the intracellular component L7/L12 as the Neisseria gonorrhoeae-derived antigens. Accordingly, exposing L7/L12 of Neisseria gonorrhoeae to the outside of the bacterial cell membrane improves detection sensitivity. Therefore, prior to contacting the sample with the first and second antibodies of the present invention, the sample may be treated to lyse the bacteria. Examples of such bacterial lysis treatments include, but are not limited to, lysis treatments using surfactants, lysing enzymes, or alkaline lysing solutions. Examples of surfactants that can be used in the lysis process include: non-ionic surfactants such as Triton X-100, Tween 20, Brij 35, Nonidet P-40, dodecyl-β-D-maltoside, octyl -β-D-glucoside, and polyoxyethylene polyoxypropylene cetylether; zwitterionic surfactants such as Zwittergent 3-12, CHAPS (3-(3-cholamidopropyl)dimethylammonio-1-propanesulfonate); and anionic surfactants such as SDS (sodium dodecyl sulfate) and sodium cholate. Examples of lysogenic enzymes that can be used in the lysis process include lysozyme, lysostaphin, pepsin, glucosidase, galactosidase, achromopeptidase, and β-N-acetyl glucosaminase.

Each step of the Neisseria gonorrhoeae test method of the present invention will be explained in detail below. In step (Ia-1), the sample is made into contact with the detection antibody to thereby label the Neisseria gonorrhoeae-derived antigen in the sample based on an antigen-antibody reaction between the detection antibody and the Neisseria gonorrhoeae-derived antigen. The method for making the sample into contact with the detection antibody is not restricted, but can typically be achieved by introducing the sample prepared as an aqueous sample into a member area impregnated with the detection antibody and maintaining it for a certain period of time. The specific embodiment varies depending on, e.g., the mode of capture in step (a). According to an example, the Neisseria gonorrhoeae-derived antigen in the sample is captured by the capture antibody immobilized on a porous membrane as a solid-phase carrier, and an aqueous reagent containing the detection antibody is introduced into the porous membrane and allowed to pass through it, whereby the detection antibody is bound to the Neisseria gonorrhoeae-derived antigen captured by the capture antibody on the porous membrane. According to another example, when the Neisseria gonorrhoeae-derived antigen in the sample is captured by the capture antibody immobilized in an area on the flow channel as a solid-phase carrier, and an aqueous reagent containing the detection antibody is passed through the flow channel, whereby the detection antibody is bind to the Neisseria gonorrhoeae-derived antigen captured by the capture antibody on the flow channel.

In step (Ia-2), the sample containing the Neisseria gonorrhoeae-derived antigen labeled with the detection antibody is made into contact with the capture antibody to thereby capture the Neisseria gonorrhoeae-derived antigen in the sample based on an antigen-antibody reaction between the capture antibody and a complex of the Neisseria gonorrhoeae-derived antigen and the detection antibody. The method for making the sample into contact with the capture antibody is not restricted, but can typically be achieved by introducing the sample prepared as an aqueous sample into the area where the capture antibody is present, and maintaining it for a certain period of time. The specific embodiment varies depending on, e.g., the solid-phase carrier to which the capture antibody is immobilized. According to an example, a porous membrane is used as a solid-phase carrier, and the sample is introduced into the porous membrane on which the capture antibody is immobilized and allowed to pass through it, whereby the Neisseria gonorrhoeae-derived antigen is captured by the capture antibody immobilized on the porous membrane. According to another example, the capture antibody is immobilized in an area on a flow channel as a solid-phase carrier, and the sample is passed through the flow channel, whereby the Neisseria gonorrhoeae-derived antigen is captured by the capture antibody immobilized in the area on the flow channel.

In step (II), the Neisseria gonorrhoeae-derived antigen to be detected is captured by the capture antibody and labeled by the detection antibody, and then is detected by using its detection label. The detection method is not restricted and can be selected according to the type of label for detection. For example, if a metallic colloid such as gold colloid is used as a detection label, the presence or absence, or the amount, of gold colloids bound to the Neisseria gonorrhoeae-derived antigen can be detected by any method such as visual inspection or camera.

The Neisseria gonorrhoeae detection method of the present invention described above may have an extremely high sensitivity, with a detection limit of Neisseria gonorrhoeae in a sample of preferably 5e4cfu/mL or less, more preferably 3e4cfu/mL or less, still more preferably 2e4cfu/mL or less (see Examples 3, 4, and 6 below). Therefore, the test can be performed using the target's urine as the sample, without the need to obtain an intra-urethral sample with a high concentration of Neisseria gonorrhoeae as was necessary for Neisseria gonorrhoeae detection kits such as immunochromatography (Non-Patent Literature 1), which significantly reduces the burden on the subject for sample acquisition. The lower limit of detection of Neisseria gonorrhoeae in the sample is not restricted, but it is usually possible to detect very low amounts of Neisseria gonorrhoeae, for example, at a concentration of approximately 1e4 cfu/mL.

It is known that urine and oral fluid have extremely complex compositions and have a wide range of pH conditions (the pH of urine is approximately 4.5 to 8, and the pH of oral fluid is approximately 4 to 7). According to the Neisseria gonorrhoeae detection method of the present invention, in its preferred embodiment, it is possible to detect Neisseria gonorrhoeae stably in samples with a wide range of pH conditions or in simulated urine samples (see Examples 5 and 6 below).

Among other things, according to an embodiment of the Neisseria gonorrhoeae detection method of the present invention, the use of a specific combination of antibodies as the combination of the capture antibody and the labeling antibody allows for more reliable diagnosis, as it can be used under a wide range of pH conditions in addition to high detection sensitivity. The pH range for the Neisseria gonorrhoeae detection method according to this embodiment may be, although is not limited to, within the range of typically pH 4.0 or more, particularly pH 4.5 or more, more particularly pH 5.8 or more, and typically pH 9.0 or less, particularly pH 8.7 or less.

The combination of antibodies for the capture antibody and the labeling antibody that enables such a wide range of pH applicability is not limited, but may preferably be, although is not limited to, either the combination (1) or (2) below.
(1) The combination of the antibody of (a) below as the capture antibody and the antibody of (c) below as the labeling antibody.
(2) The combination of the antibody of (a) below as the labeling antibody and the antibody of (c) below as the capture antibody.
   (a) An antibody having, as the heavy chain variable region sequence, an amino acid sequence having a homology of 80% or more to the amino acid sequence of SEQ ID NO:11 and, as the light chain variable region sequence, an amino acid sequence having a homology of 80% or more to the amino acid sequence of SEQ ID NO:12.
   (c) An antibody having, as the heavy chain variable region sequence, an amino acid sequence having a homology of 80% or more to the amino acid sequence of SEQ ID NO:7 and, as the light chain variable region sequence, an amino acid sequence having a homology of 80% or more to the amino acid sequence of SEQ ID NO:8.

According to the preferred embodiment of the Neisseria gonorrhoeae detection method of the present invention, it is possible to detect Neisseria gonorrhoeae in the sample with distinguishing it from Neisseria meningitidis, which belongs to the same genus Neisseria (see, e.g., Examples 1 and 2 below). Since Neisseria gonorrhoeae and Neisseria meningitidis have different drug resistance profile, the ability to distinguish them and detect them *on site* (at the clinic) is highly beneficial for early selection of appropriate drug therapy according to the bacterial species.

### [IV. Neisseria gonorrhoeae detection kit]

Another embodiment of the present invention relates to a kit for detecting Neisseria gonorrhoeae in a sample which can be used in the Neisseria gonorrhoeae detection method of the present invention (the Neisseria gonorrhoeae detection kit of the present invention).

The Neisseria gonorrhoeae detection kit of the present invention contains the capture antibody and the detection antibody explained above. The capture antibody is usually provided in an appropriate form for the type of solid-phase carrier (e.g., a vessel containing a porous membrane or a vessel containing a flow channel). The detection antibody is typically provided in the form of an aqueous reagent, which contains the detection antibody in an aqueous medium, or a dried reagent, in which the detection antibody is dried.

The kit of the present invention includes, in addition to the capture antibody and the detection antibody explained above, one or more reagents and a detection device or components thereof necessary to perform the method of the present invention using these antibodies, and/or instructions describing the procedure for performing the method of the present invention. The type of such reagents and instructions, as well as other components included in the kit of the present invention, may be determined according to the specific immunological assay used to detect bacteria of plural genera.

When the kit of the present invention contains a device for detection or components thereof, the device assembled from the kit is a device equipped with components necessary for performing the method of the present invention using the first and/or second antibody of the present invention (hereinafter also referred to as "the device of the present invention"). The specific components of the device of the present invention may be determined according to the type of immunological assay as a specific embodiment of the method of the present invention. As explained above, examples of immunological assay methods using two or more antibodies include, although are not limited to, various known immunological assays such as ELISA using antibody-loaded microtiter plates; latex particle agglutination assay using latex particles (e.g. polystyrene latex particles) loaded with antibodies; immunochromatography using antibody-loaded membranes; and sandwich assay using a detection antibody labeled with colored or chromogenic particles, enzymes or fluorophores, etc., and a capture antibody immobilized on a solid phase carrier such as magnetic particles. Devices equipped with components necessary to perform such various immunological assays may be used as the device of the present invention.

Specific examples of devices include devices of the lateral-flow type and those of the flow-through type. According to the lateral-flow devices, the analyte to be detected and the antibody to be detected are deployed parallel onto a membrane having a detection area on the surface of which the capture antibody is immobilized, and the target substance captured in the detection area of the membrane is detected. On the other hand, according to the flow-through devices, the analyte to be detected and the detection antibody are passed vertically through a membrane on which the capture antibody is immobilized on the surface, and the target substance captured on the membrane surface is detected. The method of the present invention can be applied to both lateral-flow devices and flow-through devices.

Both lateral-flow type and flow-through type immunochromatographic detection devices are well known, and the details of such devices can be designed by those skilled in the art based on their technical knowledge, other than those described herein. The following is a description of the schematic configuration of the detection mechanism of the lateral flow type immunochromatographic detection device, with reference to the drawings. The schematic configuration of the detection mechanism of the lateral-flow type immunochromatography detection system will be described below with reference to the figure. However, these are only examples of the schematic configuration of the detection procedure, and the configuration of the lateral-flow type immunochromatographic detection system is not limited in any way to the embodiment illustrated in the figure.

Figure 3 is a cross-sectional view of a schematic configuration of a strip-shaped detection mechanism, which is an example of a detection mechanism of a lateral-flow type immunochromatographic detection system. The detection mechanism (10) of Figure 3 contains an insoluble membrane carrier (1) for chromatographic development; a strip-shaped member (conjugate pad) (2) (which is impregnated with the detection antibody) and a sample addition member (sample pad) (3) arranged at one end of the lengthwise direction of the strip (upstream of the sample flow B) on the insoluble membrane carrier (1); and a member for absorption (absorption pad) (4) arranged at the other end (downstream side of the sample flow B) at the other end of the lengthwise direction of the strip (downstream of the sample flow B) on the insoluble membrane carrier (1). Arranged in the center of the strip lengthwise direction on the insoluble membrane carrier (1) is a site (5) on which the capture antibody is immobilized, along with, if necessary, a site (6) on which the control reagent is immobilized. The control reagent is a reagent that does not bind to the analyte but does bind to the detection antibody.

At the time of use, when a sample A is applied on the sample addition member (sample pad) (3), the sample A passes through the member (conjugate pad) (2) impregnated with the detection antibody, and flows through the insoluble membrane carrier (1) in the sample flow direction A. During this process, the analyte in the sample (in the case of the present invention, bacteria to be detected) binds to the detection antibody to form an analyte-detection antibody complex. When the sample A passes through the capture-antibody immobilized site (5), the analyte in the sample binds to the capture antibody to form a capture antibody-analyte-detection antibody complex. When the sample A passes through the control-reagent immobilized site (7), the detection antibody that has not bound to the analyte binds to the control reagent (7), whereby it is confirmed that the test has been completed (i.e., that sample A has passed through the capture antibody (6)). The presence or amount of the analyte can be detected by detecting the label of the detection antibody in the capture antibody-analyte-detection antibody complex that exists in the capture-antibody immobilized site (6) by known means. If necessary, the label of the detection antibody may be sensitized by known methods to facilitate detection.

The detection antibody-impregnated member (conjugate pad) (2), the sample addition member (sample pad) (3), and/or the control reagent-immobilized site (7) may be optionally omitted. When the present mechanism lacks the detection antibody-impregnated member (conjugate pad) (2), the same test as above can be performed by applying the sample A and the detection antibody to one end on the insoluble membrane carrier (1) in a pre-mixed state or separately, either simultaneously or sequentially.

Even when the capture antibody and the detection antibody are interchanged, a detection kit can be constructed to enable the same detection.

Although the method of manufacturing the kit of the present invention described above is not limited, it is preferable to manufacture the kit by a manufacturing method that includes at least the step of immobilizing the capture antibody to a carrier having a detection region at the detection region.

Specifically, when the kit is an immunochromatography kit, it is preferable to manufacture the kit by a production method that includes at least the steps of: immobilizing the capture antibody to a carrier having a detection region at the detection region; attaching the detection antibody to a conjugate pad; and placing the conjugate pad upstream of the detection region on the carrier.

The production method of the kit of the present invention may preferably further include the steps of: adding the detection label to one of the first and second monoclonal antibodies to thereby prepare the detection antibody; and immobilizing the other of the first and second monoclonal antibodies as the capture antibody to the detection region of the carrier. The first and second monoclonal antibodies have been described in detail previously.

Specifically, when an antibody which causes an antigen-antibody reaction with an epitope containing one or more amino acid residues selected from the 2nd to 14th amino acid residues from the N-terminal of the amino acid sequence of Neisseria gonorrhoeae L7/L12 shown in SEQ ID NO:1 is to be used as the first monoclonal antibody, it may be preferred to obtain the first monoclonal antibody by inoculating an animal with an epitope polypeptide containing such an epitope, obtaining an antiserum containing antibodies that produce an antigen-antibody reaction with the epitope polypeptide, and purifying and fractionating the antibody from the antiserum. The method for obtaining the antibody can be carried out either separately from the production method of the kit of the present invention or as a step of the production method of the kit of the present invention.

On the other hand, when an antibody which causes an antigen-antibody reaction with an epitope containing one or more amino acid residues selected from the 102nd to 123rd amino acid residues from the N-terminal of the amino acid sequence of Neisseria gonorrhoeae L7/L12 shown in SEQ ID NO:1 is used as the second monoclonal antibody, it may be preferred to obtain the second monoclonal antibody by inoculating an animal with an epitope polypeptide containing such an epitope, obtaining an antiserum containing antibodies that produce an antigen-antibody reaction with the epitope polypeptide, and purifying and fractionating the antibody from the antiserum. The method for obtaining the antibody can be carried out either separately from the production method of the kit of the present invention or as a step of the production method of the kit of the present invention.

Alternatively, an antibody with a known amino acid sequence(s) of the heavy chain variable region and/or the light chain variable region may be used as the first and/or second monoclonal antibody to make the kit. The amino acid sequence(s) of the heavy chain variable region and/or the light chain variable region of the first and/or second monoclonal antibody have been described in detail above.

As explained above, either the first and second monoclonal antibodies may be the capture antibody and either may be a labeling antibody.

Specifically, the combination of the capture antibody and the labeling antibody used in the Neisseria gonorrhoeae detection method of the present invention may be, although is not limited to, either the combination (1) or (2) below.
(1) The combination of the antibody of (a) or (b) below as the capture antibody and the antibody of (c) below as the labeling antibody.
(2) The combination of the antibody of (a) or (b) below as the labeling antibody and the antibody of (c) below as the capture antibody.
   (a) An antibody having, as the heavy chain variable region sequence, an amino acid sequence having a homology of 80% or more to the amino acid sequence of SEQ ID NO:11 and, as the light chain variable region sequence, an amino acid sequence having a homology of 80% or more to the amino acid sequence of SEQ ID NO:12.
   (b) An antibody having, as the heavy chain variable region sequence, an amino acid sequence having a homology of 80% or more to the amino acid sequence of SEQ ID NO:9 and, as the light chain variable region sequence, an amino acid sequence having a homology of 80% or more to the amino acid sequence of SEQ ID NO:10.
   (c) An antibody having, as the heavy chain variable region sequence, an amino acid sequence having a homology of 80% or more to the amino acid sequence of SEQ ID NO:7 and, as the light chain variable region sequence, an amino acid sequence having a homology of 80% or more to the amino acid sequence of SEQ ID NO:8.

Among them, from the viewpoint of achieving applicability to a wide range of pH conditions as described below, the combination of the capture antibody and labeling antibody may preferably be, although is not limited to, either the combination (1) or (2) below.
(1) The combination of the antibody of (a) below as the capture antibody and the antibody of (c) below as the labeling antibody.
(2) The combination of the antibody of (a) below as the labeling antibody and the antibody of (c) below as the capture antibody.
   (a) An antibody having, as the heavy chain variable region sequence, an amino acid sequence having a homology of 80% or more to the amino acid sequence of SEQ ID NO:11 and, as the light chain variable region sequence, an amino acid sequence having a homology of 80% or more to the amino acid sequence of SEQ ID NO:12.
   (c) An antibody having, as the heavy chain variable region sequence, an amino acid sequence having a homology of 80% or more to the amino acid sequence of SEQ ID NO:7 and, as the light chain variable region sequence, an amino acid sequence having a homology of 80% or more to the amino acid sequence of SEQ ID NO:8.

### EXAMPLES

The present invention will be described in more detail with reference to the examples below. However, the present invention is not bound by the following examples, and can be implemented in any form within the scope that does not depart from the purpose of the present invention.

### [Example 1: Production and screening of antibodies]

Antibodies against ribosomal protein L7/L12 of Neisseria gonorrhoeae were produced by the method described in WO2001/057089 A. Specifically, polypeptides 1 to 4, which each have all or a part of the amino acid sequence of L7/L12 of Neisseria gonorrhoeae, were used as antigen polypeptides derived from Neisseria gonorrhoeae. Polypeptide 5, which has all of the L7/L12 amino acid sequence of Neisseria meningitidis, was used as a similar antigen polypeptide to verify antibody cross-reactivity.

The alignment of the L7/L12 amino acid sequence of Neisseria gonorrhoeae with the amino acid sequences of polypeptides 1 to 5 is shown in Figure 2, and the SEQ ID NOs of these amino acid sequences and the correspondence between these sequences are shown in Table 1 below. In the table, "Yes" indicates that the polypeptide has a partial sequence consisting of the relevant amino acid residues of Neisseria gonorrhoeae L7/L12, and "No" indicates that the polypeptide lacks such a sequence. The amino acid sequences of Neisseria gonorrhoeae L7/L12 and Neisseria meningitidis L7/L12 differ only in that the 115th amino acid residue from the N-terminus is alanine in Neisseria gonorrhoeae, while it is glutamic acid in Neisseria meningitidis. Accordingly, in the table below, the column of polypeptide 5 corresponding to amino acid residues 102 to 123 of Neisseria meningitidis L7/L12 is indicated as "Similar."

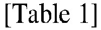

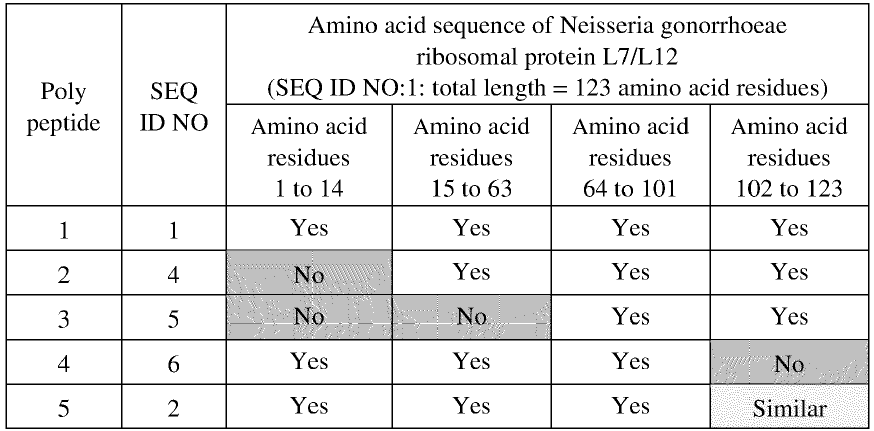

The above polypeptides 1 to 5 were prepared by the following procedure. Expression vectors incorporating DNA encoding each of the polypeptides 1 to 5 were prepared, and E. coli were transformed using these expression vectors. The transformed E. coli were cultured in LB medium, and the polypeptides 1 to 5 expressed from the transformed bacteria and released into the culture medium were purified as fusion proteins by affinity columns using the tag sequences derived from the expression vectors.

Polypeptide 1 obtained by the above procedure was inoculated into mice with each adjuvant. Thirty days after inoculation, spleen cells of the mice were harvested and fused with myeloma cells to generate antibody-producing hybridomas.

Monoclonal antibodies produced from hybridomas were sorted using ELISA and other methods. The aforementioned polypeptides 1 to 5 were used for selection. Specifically, the aforementioned polypeptides 1 to 5 were solid-phased in a 96-well microplate at a concentration of 2 µg/mL each in 1 × PBS, reacted with each monoclonal antibody obtained above at a concentration of 100 ng/mL, reacted with HRP (horseradish peroxidase)-labeled anti-IgG antibody, and finally mixed with a substrate to produce a color, whereby the reaction pattern of each monoclonal antibody against polypeptides 1 to 5 was evaluated.

The above procedure was used to obtain the final six monoclonal antibodies. The reactivity of the six monoclonal antibodies obtained against polypeptides 1 to 5 are shown in Table 2 below. In the table, those with an absorbance of 0.1 or greater at a wavelength of 450 nm are indicated with "+" and those with an absorbance of less than 0.1 are indicated with "--."

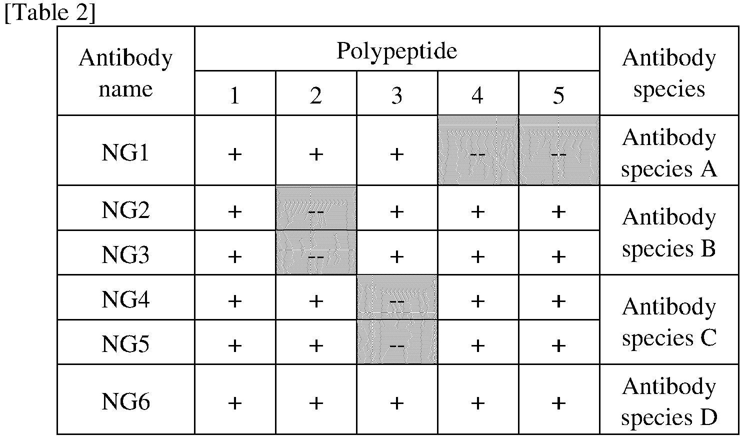

Based on the above reaction patterns, the six monoclonal antibodies were classified into the following four species.
*Antibody species A (NG1): This antibody species does not react with polypeptide 4 and polypeptide 5, indicating that it reacts with a partial sequence consisting of the 102nd to 123rd amino acid residues from the N-terminal, including the 115th amino acid from the N-terminal of Neisseria gonorrhoeae L7/L12.
*Antibody species B (NG2, NG3): This antibody species does not react with polypeptide 2, indicating that it reacts with a partial sequence consisting of the 2nd to 14th amino acid residues from the N-terminal of Neisseria gonorrhoeae L7/L12.
*Antibody species C (NG4, NG5): This antibody species reacts with polypeptide 2 but not with polypeptide 3, indicating that it reacts with a partial sequence consisting of the 15th to 63rd amino acid residues from the N-terminal of Neisseria gonorrhoeae L7/L12.
*Antibody species D (NG6): This antibody species reacts with all of polypeptides 1 to 5, indicating that it reacts with a partial sequence consisting of the 64th to 101st amino acid residues from the N-terminal of Neisseria gonorrhoeae L7/L12.

Among the six monoclonal antibodies obtained, Antibody NG1, which belongs to Antibody species A, and Antibodies NG2 and NG3, which belong to Antibody species B, were analyzed for their heavy chain and light chain variable region sequences by the usual method. The amino acid sequences of the heavy and light chain variable regions of Antibody NG1 are shown in SEQ ID NO:7 and SEQ ID NO:8, respectively. The amino acid sequences of the heavy and light chain variable regions of Antibody NG2 are shown in SEQ ID NO:9 and SEQ ID NO:10, respectively. The amino acid sequences of the heavy and light chain variable regions of Antibody NG3 are shown in SEQ ID NO:11 and SEQ ID NO:12, respectively.

### [Example 2: Evaluation of the performance of antibody combinations in detecting

### Neisseria gonorrhoeae by ELISA]

Next, various combinations of antibodies of Antibody species A to D were used as capture antibodies and detection antibodies, and the performance of each combination in detecting Neisseria gonorrhoeae was evaluated by ELISA. Antibody NG1 was used as a representaive example for Antibody species A, Antibody NG3 for Antibody species B, Antibody NG5 for Antibody species C, and Antibody NG6 for Antibody species D. When each of Antibody species A to D was used as the detection antibody, HRP (horseradish peroxidase) labeling was added according to the usual method before use.

First, Neisseria gonorrhoeae (ATCC 700825) and Neisseria meningitidis (ATCC 13090) were each seeded on chocolate agar medium, incubated at 37°C and 5 to 10% CO₂ for 24 hours, and the colonies generated were collected and suspended in saline. The absorbance of the suspended saline solution was measured with a spectrophotometer (wavelength 600 nm), and the concentration at which the absorbance reached 2 was estimated to contain 1e9 cfu/mL of Neisseria gonorrhoeae, which was used to assign a bacterial number to the saline suspension of Neisseria gonorrhoeae. The saline suspensions of Neisseria gonorrhoeae and Neisseria meningitidis were serially diluted with saline to obtain a saline suspension of Neisseria gonorrhoeae with a bacterial concentration of 6e6 cfu/mL.

In addition, an extract solution with the following compositions was prepared.
- 0.3% TritonX-100
- 0.3% Polyoxyethylene polyoxypropylene cetylether
- 0.2% Bovine serum albumin
- 0.1M Sodium chloride

One hundred µL of the Neisseria gonorrhoeae saline suspension with a bacterial concentration of 6e6 cfu/mL as described above was combined with 500 µL of the extract solution described above, mixed well, and allowed to stand for 1 hour to prepare an extracted sample solution with a bacterial concentration of 1e6 cfu/mL.

Fifty µL of a capture antibody solution, in which a capture antibody selected from Antibody species A to D was dissolved in 1 × PBS at a concentration of 10 µg/mL, was introduced into each well of a 96-well microplate and allowed to stand at 4°C for 16 hours to immobilize the capture antibody on the bottom of the well. After the capture antibody solution remaining in each well was then removed, the well was washed with 1 × PBS containing 0.05% Tween 20, and 200 µL of a solution of BSA dissolved in 1 × PBS at 1% concentration was introduced into each well, and allowed to react for 2 hours to block the well. Next, 50 µL of the extracted sample solution of Neisseria gonorrhoeae or Neisseria meningitidis with a bacterial concentration of 1e6cfu/mL prepared above was injected into each well, and allowed to react for 1 hour. After the extracted sample solution remaining in each well was removed, the well was washed with 1 × PBS containing 0.05% Tween 20, and 50 µL of an HRP-labeled detection antibody selected from Antibody species A to D dissolved in 1 × PBS at a concentration of 1 µg/mL was introduced into each well, and allowed to react for 1 hour. After the detection antibody solution remaining in each well was removed, the well was washed with 1 × PBS containing 0.05% Tween 20, and 100 µL of luminescent substrate was added to each well, and allowed to stand for 30 minutes. 100 µL of 1N HCl was added to each well as stopping solution to stop the color reaction. Finally, the absorbance (wavelength 450 nm) of each well was measured with a plate reader (Molecular Devices "Spectra Max 190").

The results are shown in Table 3 below. In the table, antibody combinations in which the difference between the absorbance of the well reacted with the extracted sample solution of Neisseria gonorrhoeae and the absorbance of the well reacted with the extracted sample solution containing neither Neisseria gonorrhoeae nor Neisseria meningitidis (Absorbance difference A) was less than 1.0 are indicated with "A: -." Of the combinations in which Absorbance difference A described above was 1.0 or greater, antibody combinations in which the difference between the absorbance of the well reacted with the extracted sample solution containing Neisseria meningitidis and the absorbance of the well reacted with the extracted sample solution containing neither Neisseria gonorrhoeae nor Neisseria meningitidis (Absorbance difference B) was less than 1.0 are indicated with "A: +, B: -," and antibody combinations in which both Absorbance differences A and B described above were both greater than 1.0 are indicated with "A: +, B: +."

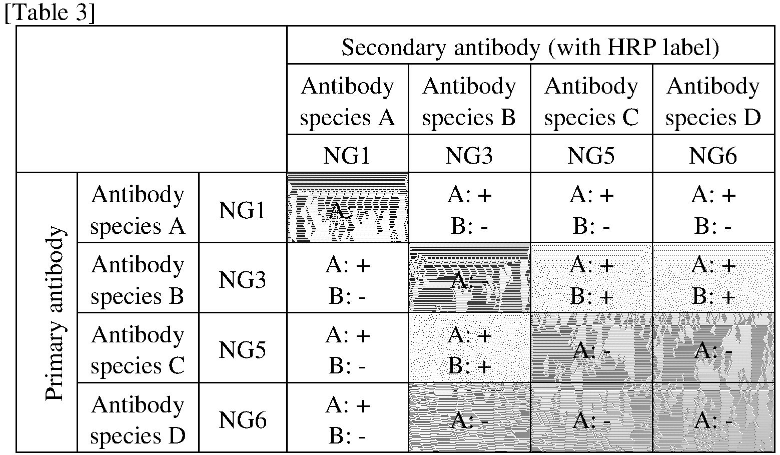

These results show that all the antibody combinations containing one Antibody species A (NG1) have a clear difference in signal between the extracted sample solution containing Neisseria gonorrhoeae and the extracted sample solution containing Neisseria meningitidis. It is therefore deemed that Antibody species A generates an antigen-antibody reaction with the partial sequence of Neisseria gonorrhoeae ribosomal protein L7/L12 including the 115th amino acid residue from the N-terminus, which is the only difference between the L7/L12 amino acid sequence of Neisseria gonorrhoeae ribosomal protein and that of Neisseria meningitidis ribosomal protein.

### [Example 3: Evaluation of the detection sensitivity of Neisseria gonorrhoeae by immunochromatography device]

Next, immunochromatography systems for Neisseria gonorrhoeae detection were produced using an antibody selected from Antibody species B to D as the capture antibody and an antibody of Antibody species A as the detection antibody, and the performance of the immunochromatography systems for Neisseria gonorrhoeae detection were verified. Antibody NG1 was used as a representative for Antibody species A for evaluation. When an antibody from Antibody species B to D was used as the detection antibody, HRP labeling was added to the antibody according to the usual method before use.

First, the monoclonal antibodies of Antibody species B, C, and D were each dissolved in 50 mM sodium phosphate buffer to 1.5 mg/mL with 3% (v/v) of trehalose. The resulting antibody solutions were each applied to a commercially available nitrocellulose membrane cut into a square of 2.5 cm width and 30 cm length at a volume of 1 µL per cm length, and then dried to prepare a membrane carrier for chromatographic development impregnated with the antibody from Antibody species B, C, or D as the capture antibody.

Solution of gold colloids (60 nm particle size) was combined and mixed with 1/10 volume of 0.1 M sensitizing buffer (TAPSO, pH 7.0, by Dojin Chemical Laboratory Co., Ltd.) and the antibody from Antibody species A against ribosomal protein L7/L12 of Neisseria gonorrhoeae, and allowed to stand at room temperature for 30 minutes to bind the antibody to the surface of gold colloid particles. 2.5% casein solution was added for blocking, to thereby prepare a gold colloid-labeled antibody solution with a final concentration of 0.2%. The solution was soaked into commercially available glass fiber sheets and dried overnight in a drying chamber with a humidity of 0 to 60% to thereby prepare labeling antibody-impregnated members, which were impregnated with the gold colloid-labeled antibody from Antibody species A as the detection antibody.

The membrane carrier for chromatographic development (1) and the gold colloid-labeled antibody-impregnated component (2) prepared by the procedure described above were used in combination with a cotton cloth as the sample addition member (3) and a paper filter as the absorption component (4). These components were laminated on a substrate (5) and cut into 5 mm widths, whereby an immunochromatography device having the structure shown in Figure 3 was produced.

Saline suspension of Neisseria gonorrhoeae was diluted stepwise with saline to obtain saline suspensions of Neisseria gonorrhoeae with bacterial concentrations of 1e5, 5e4, 2e4, and 1e4 cfu/mL. 100 µL of each of these Neisseria gonorrhoeae saline suspensions was combined and mixed with 500 µL of the extract solution, and allowed to stand for 1 hour to prepare the extracted sample solution.

Each of the extracted sample solutions prepared above was dropped in a volume of 115 µL into each immunochromatography system for Neisseria gonorrhoeae detection prepared according to the procedure described above, and the results were visually determined after 15 minutes. The results are shown in Table 4 below. In the table, "-" indicates a visual positive result and "+" indicates a visual negative result.

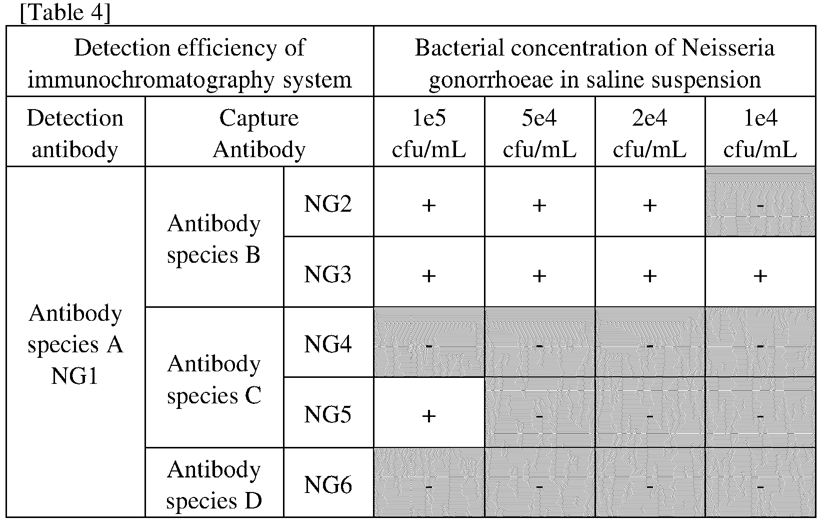

According to these results, the combination of Antibody species A and Antibody species B showed good detection performance, and was able to detect Neisseria gonorrhoeae at concentrations as low as 1 to 2e4 cfu/mL (in the amount of bacteria before extraction). On the other hand, the combination of Antibody species A with Antibody species C or D could not detect Neisseria gonorrhoeae at concentrations of less than 1e5 cfu/mL (of bacteria before extraction).

### [Example 4: Evaluation of the detection sensitivity of Neisseria gonorrhoeae by ELISAI

The antibody combinations that enabled detection of Neisseria gonorrhoeae from samples with concentrations as low as 1e5 cfu/mL in Example 3 (Evaluation of the detection sensitivity of Neisseria gonorrhoeae by immunochromatography device) were then subjected to evaluation for the detection performance by ELISA, which is an example of an immunoassay principle different from immunochromatography.

Fifty microliters of capture antibody solution, in which the capture antibody (Antibody NG2 or NG3 from Antibody species B or Antibody NG5 from Antibody species C) was dissolved in 1 ×PBS at a concentration of 10 µg/mL, introduced into each well of the 96-well microplate, and allowed to stand at 4°C for 16 hours, whereby the capture antibody was immobilized on the bottom of the well. After the capture antibody solution remaining in each well was removed, the well was washed with 1 × PBS containing 0.05% Tween 20, and 200 µL of a solution of BSA dissolved in 1 × PBS at 1% concentration was introduced into each well, and allowed to react for 2 hours to block the well. 50 µL of each of the extracted sample solutions with bacterial concentrations of 1e5, 5e4, 2e4, and 1e4 cfu/mL prepared by the method described above was injected into each well and allowed to react for 1 hour. After the extracted sample solution remaining in each well was removed, the well was washed with 1× PBS containing 0.05% Tween 20, and 50 µL of a detection antibody solution containing the HRP-labeled detection antibody (NG1) dissolved in 1× PBS at a concentration of 1 µg/mL was introduced and allowed to react for 1 hour. After the detection antibody solution remaining in each well was removed, the well was washed with 1 ×PBS containing 0.05% Tween 20, and 100 µL of luminescent substrate was added to each well, and allowed to stand for 30 minutes. 100 µL of 1 provisional HCl was added to each well as stopping solution to stop the color reaction. Finally, the absorbance (wavelength 450 nm) of each well was measured with a plate reader (Molecular Devices "Spectra Max 190").

The results are shown in Table 5 below. In the table, "+" indicates that the difference between the absorbance of the well reacted with the extracted sample solution at each bacterial concentration and the absorbance of the well reacted with the extracted sample solution without bacteria was 0.1 or greater, and "-" indicates that the difference was less than 0.1.

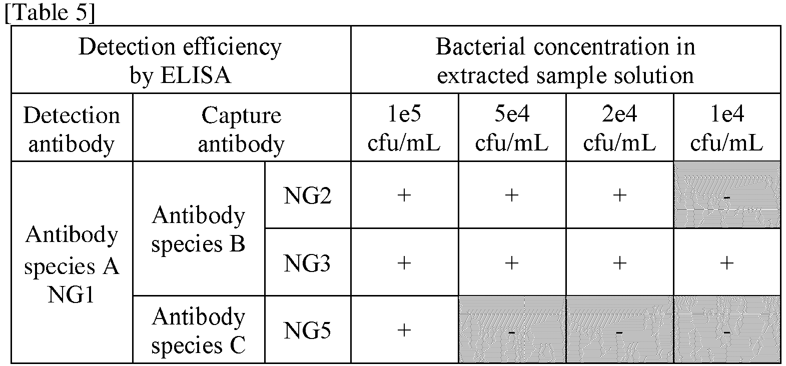

According to these results, the combination of Antibody species A with Antibody species B showed good detection performance by ELISA, and was able to detect Neisseria gonorrhoeae at concentrations as low as 1 to 2e4 cfu/mL (in the amount of bacteria before extraction). On the other hand, the combination of Antibody species A with Antibody species C failed to detect Neisseria gonorrhoeae at concentrations of less than 1e5 cfu/mL (in the amount of bacteria before extraction).

### [Example 5: Evaluation of the detection sensitivity of Neisseria gonorrhoeae under a wide pH range by ELISA]

The combination that was able to detect Neisseria gonorrhoeae from samples with a bacterial concentration of 1e5 cfu/mL in Example 3 (Evaluation of the detection sensitivity of Neisseria gonorrhoeae by immunochromatography device) was also subjected to evaluation for their immunodetection performance over a wide pH range using ELISA.

The following three buffer solutions for immunoreaction were prepared for evaluation.
*Solution 1: 10mM K₂ HPO₄, 90mM KH₂ PO₄, 100mM NaCl, pH 5.8, conductivity 18mS/cm
*Solution 2: 60mM K₂ HPO₄, 40mM KH₂ PO₄, 100mM NaCl, pH 6.8, conductivity 20mS/cm
*Solution 3: 100mM K₂ HPO₄, 100mM NaCl, pH 8.7, conductivity 23mS/cm
*Solution 4: 100mM KH₂ PO₄, 100mM NaCl, pH 4.5, conductivity 17mS/cm

Fifty µL of each of the capture antibody solutions (NG2, NG3, and NG5), in which each capture antibody was dissolved in 1×PBS at a concentration of 10 µg/mL, was introduced into each well of the 96-well microplate and allowed to stand at 4°C for 16 hours to immobilize the capture antibody on the bottom of the well. After the capture antibody solution remaining in each well was removed, the well was washed with 1× PBS containing 0.05% Tween 20, and 200 µL of BSA dissolved in 1× PBS at 1% concentration was introduced into each well, and allowed to react for 2 hours for blocking. Next, each of the extracted sample solutions with a bacterial concentration of 1.6e7cfu/mL (for addition to Solutions 1, 2, and 3) or 3e7cfu/mL (for addition to Solution 4) prepared above was added to the immunoreaction buffer (Solutions 1, 2, 3, and 4) in 1/100 volume, whereby a pH-adjusted sample solution containing Neisseria gonorrhoeae at a bacterial concentration of 1.6e5cfu/mL (Solutions 1, 2, and 3) or 3e5cfu/mL (Solution 4) was obtained. 50 µL of this pH-adjusted sample solution was injected into each well, and allowed to react for 1 hour. After the pH-adjusted sample solution remaining in each well was removed, the well was washed with 1× PBS containing 0.05% Tween 20, and 50 µL of each pH-adjusted detection antibody solution, in which the HRP-labeled detection antibody (NG1) was dissolved at a concentration of 1 µg/mL in each of the immunoreaction buffers (Solutions 1, 2, and 3) described above, was introduced into each well, and reacted for 1 hour. In this step, the pH of the pH-adjusted sample solution to be placed in each well was aligned with the pH of the pH-adjusted detection antibody solution. After the pH-adjusted detection antibody solution remaining in each well was removed, the well was washed with 1×PBS containing 0.05% Tween 20, and 100 µL of luminescent substrate was introduced into each well, and allowed to stand for 30 minutes. The color reaction was stopped by adding 100 µL of 1 provisional HCl as a stopping solution. Finally, the absorbance (wavelength 450 nm) of each well was measured with a plate reader (Molecular Devices "Spectra Max 190").

The results are shown in Table 6 below. In the table, "++" indicates that the difference between the absorbance of the well reacted with the extracted sample solution at each bacterial concentration and the absorbance of the well reacted with the extracted sample solution without bacteria was 1.0 or more, "+" indicates that the difference was 0.5 or more but less than 1.0, and "-" indicates that the difference was less than 0.5.

**[Table 6]**

| | | | Wide pH range applicability (ELISA) | | |
|---|---|---|---|---|---|
| Detection antibody | Capture antibody | | pH 5.8 | pH 6.8 | pH 8.7 |
| Antibody species A NG1 | Antibody species B | NG2 | ++ | ++ | + |
| | | NG3 | ++ | ++ | ++ |
| | Antibody species C | NG5 | ++ | ++ | - |

The combination of Antibody species A and Antibody species B was capable of stable immunodetection over a wide pH range of pH 5.8 to 8.7, and is therefore considered suitable for detection of Neisseria gonorrhoeae from various samples, including urine and oral samples whose pH can vary over a wide range. The reason why the combination of Antibody species A and Antibody species B was stable and immunodetectable over a wide pH range of pH 5.8 to 8.7 is estimated as follows. The amino acid sequence of a part of the antigen with which Antibody species A causes an antigen-antibody reaction, i.e., the part consisting of the 102nd to 121st amino acid residues from the N-terminus in the L7/L12 amino acid sequence of Neisseria gonorrhoeae shown in SEQ ID NO:1, and the amino acid sequence of a part of the antigen with which Antibody species B causes an antigen-antibody reaction, i.e., the part consisting of the 3rd to 14th amino acid residues from the N-terminus in the amino acid sequence of L7/L12 of Neisseria gonorrhoeae shown in SEQ ID NO:1, are considered to have stable structure and chemical properties in the pH range of 5.8 to 8.7.

The combination of Antibody NG1 as Antibody species A and Antibody NG3 as Antibody species B was capable of stable immunodetection even at pH as low as 4.5. This combination is therefore considered to be particularly suitable for detecting Neisseria gonorrhoeae in various samples, such as urine and oral samples whose pH can change over a wide range. The reason why the combination of Antibodies NG1 and NG3 could stably immunodetect gonorrhea at pH as low as 4.5 is estimated to be because the antigen recognition site of Antibody species B, i.e., Antibody NG3, has a particularly stable structure and chemical properties even at low pH.

### [Example 6: Evaluation of the detection efficiency of Neisseria gonorrhoeae in urine sample]

The combinations of Antibody species A and Antibody species B, which were demonstrated to have good detection performance and wide pH range adaptability, were then subjected to evaluation for their capability to detect Neisseria gonorrhoeae in actual urine samples. Simulated urine samples were prepared by adding each of saline suspensions of Neisseria gonorrhoeae with known bacterial concentrations to commercially available donor urine (LEE BIOSOLUTIONS, pH 6.3, conductivity 10 mS/cm, confirmed negative in Neisseria gonorrhoeae nucleic acid test by Cobas 8800), and urinary Neisseria gonorrhoeae concentrations of 0 cfu/mL, 1e4cfu/mL, and 2e4cfu/mL. 100 µL of each of the simulated urine samples was combined and mixed with 500 µL of the extract solution to prepare the extracted sample solution.

Each of the extracted sample solutions prepared above was dropped by 115 µL into the immunochromatography systems for Neisseria gonorrhoeae detection prepared by the procedure described in Example 3, and the results were visually determined after 15 minutes. The results are shown in Table 7 below. In the table, "+" indicates visually positive, and "-" indicates visually negative.

**[Table 7]**

| | | | Simulated urine sample | | |
|---|---|---|---|---|---|
| Detection antibody | Capture antibody | | 2e4 cfu/mL | 1e4 cfu/mL | 0 cfu/mL |
| Antibody species A NG1 | Antibody species B | NG2 | + | - | - |
| | | NG3 | + | + | - |

The combination of Antibody species A and Antibody species B showed good detection performance even in urine samples, and was able to detect gonorrhea at concentrations as low as 1-2e4 cfu/mL (in the amount of bacteria before extraction).

### [Example 7: Evaluation of the detection sensitivity of Neisseria gonorrhoeae by immunochromatography]

The combinations of Antibody species A and Antibody species B, which were demonstrated to have good detection performance and wide pH range adaptability, were then subjected to evaluation for their ability to detect Neisseria gonorrhoeae in the specimen with distinguishing it from Neisseria meningitidis, using the immunochromatography systems produced in Example 3.

Saline suspension of Neisseria gonorrhoeae was diluted stepwise with saline to obtain saline suspensions of Neisseria gonorrhoeae at concentrations of 1.2e6, 6e5, 3e5, 1.2e5, and 6e4 cfu/mL. 100 µL of each of the saline suspensions of Neisseria gonorrhoeae was combined and mixed with 500 µL of the extract solution, and allowed to stand for 1 hour, whereby the extracted sample solutions at concentrations of 2e5, 1e5, 5e4, 2e4, and 1e4 cfu/mL were prepared.

One hundred fifteen µL of each extracted sample solutions prepared above was dropped into the immunochromatography systems for Neisseria gonorrhoeae detection produced above, and the results were visually determined after 15 minutes. The results are shown in Table 8 below. In the table, "+" indicates visually positive and "-" indicates visually negative.

**[Table 8]**

| Detection antibody (Antibody species A) | Capture antibody (Antibody species B) | Bacterial concentration in extracted sample solution | | | | | |
|---|---|---|---|---|---|---|---|
| | | Bacteria Species | 2e5 cfu/mL | 1e5 cfu/mL | 5e4 cfu/mL | 2e4 cfu/mL | 1e4 cfu/mL |
| NG1 | NG2 | Neisseria gonorrhoeae | + | + | + | + | - |
| | | Neisseria meningitidis | | | | | |
| | NG3 | Neisseria gonorrhoeae | + | + | + | + | + |
| | | Neisseria meningitidis | | | | | |

The combination of Antibody species A and Antibody species B was at least 10 times more sensitive in detecting Neisseria gonorrhoeae than Neisseria meningitidis, and was able to detect Neisseria gonorrhoeae in the samples with distinguishing it from Neisseria meningitidis.

### [Example 8: Evaluation of bacteria specificity]

The combinations of Antibody species A and Antibody species B, which were demonstrated to have good detection performance and wide pH range adaptability, were then subjected to evaluation for their lack of cross-reactivity with bacteria belonging to other genera, using the immunochromatography systems produced in Example 3.

The suspensions of each of the bacterial species listed in Table 9, with the exception of Chlamydia trachomatis, Chlamydia pneumoniae, and Mycoplasma pneumoniae, were subjected to bacterial cell count by the method described in Example 2, and then serially diluted with saline to obtain saline suspension of each bacterium with a bacterial concentration of 6e7cfu/mL. 500 µL of the extract solution was combined and mixed with 100 µL of the bacterial saline suspension and allowed to stand for 1 hour to prepare an extracted sample solution with a bacterial concentration of 1e7 cfu/mL. For Chlamydia trachomatis, 2e8 IFU/ml of inactivated bacterial solution purchased from Microbix was serially diluted with saline, and 100 µL of the resulting bacterial saline suspension was combined and mixed with 500 µL of the extract solution and allowed to stand for 1 hour, whereby an extracted sample solution with a concentration of 2e6 IFU/ml was prepared. For Chlamydia pneumoniae, a 2e8 IFU/ml inactivated bacterial solution purchased from Microbix was serially diluted with saline, and 500 µL of the extract solution was added to 100 µL of the bacterial saline suspension bacteria, mixed well, and allowed to stand for 1 hour, whereby an extracted sample solution was then prepared at a bacterial concentration of 4e6 IFU/ml. For Mycoplasma pneumoniae, 5e7 IFU/ml of inactivated bacterial solution purchased from Microbix was serially diluted with saline, and 500 µL of the extract solution was added to 100 µL of the bacterial saline suspension, mixed well, and allowed to stand for 1 hour, whereby an extracted sample solution with a bacterial concentration of 2e6 IFU/ml was thus prepared.

Each of the extracted sample solutions prepared above was dropped by 115 µL into the immunochromatography systems for Neisseria gonorrhoeae detection produced above, and the results were visually determined after 15 minutes. The results are shown in Table 9 below. In the table, "+" indicates visually positive and "-" indicates visually negative.

**[Table 9]**

| Bacteria species | | Detection antibody (Antibody species A) NG1 |
|---|---|---|
| | Capture antibody (Antibody species B) NG2 | Capture antibody (Antibody species B) NG3 |
| *Neisseria gonorrhoeae* | + | + |
| *Neisseria lactamica* | - | - |
| *Chlamydia trachomatis* | - | - |
| *Chlamydia pneumoniae* | - | - |
| *Escherichia coli* | - | - |
| *Klebsiella pneumoniae* | - | - |
| *Proteus mirabilis* | - | - |
| *Pseudomonas aeruginosa* | - | - |
| *Staphylococcus aureus* | - | - |
| *Enterococcus faecalis* | - | - |
| *Streptococcus pneumoniae* | - | - |
| *Streptococcus pyogenes* | - | - |
| *Legionella pneumophila SG1* | - | - |
| *Haemophilus influenzae* | - | - |
| *Bacillus subtilis* | - | - |
| *Moraxella catarrhalis* | - | - |
| *Mycoplasma pneumoniae* | - | - |
| *Bordetella pertussis* | - | - |
| negative sample | - | - |

The combination of Antibody species A with Antibody species B does not cause a cross-reaction with bacteria in a sample other than Neisseria gonorrhoeae as shown in Table 9.

### INDUSTRIAL APPLICABILITY

The present invention can be widely used in medical fields involving detection of Neisseria gonorrhoeae, and therefore has extremely high industrial usefulness.

### EXPLANATION OF SYMBOLS

- 10: Detection mechanism
- 1: Insoluble membrane carrier for chromatography development
- 2: Detection antibody-impregnated member (conjugate pad)
- 3: Sample addition member (sample pad)
- 4: Member for absorption (absorption pad)
- 5: Substrate
- 6: Capture antibody-immobilized site
- 7: Control reagent-immobilized site
- A: Sample
- B: Sample flow

## Claims

1. A kit for detecting Neisseria gonorrhoeae in a sample, comprising:
a capture antibody for capturing a Neisseria gonorrhoeae-derived antigen in the sample; and
a detection antibody having a detection label for labeling the Neisseria gonorrhoeae-derived antigen in the sample,
wherein Neisseria gonorrhoeae is detected via an immune reaction between the Neisseria gonorrhoeae-derived antigen in the sample, the capture antibody, and the detection antibody to form a structure sandwiching the Neisseria gonorrhoeae-derived antigen,
wherein the Neisseria gonorrhoeae-derived antigen is a ribosomal protein L7/L12 of Neisseria gonorrhoeae,
wherein one of the capture antibody and the detection antibody is a first monoclonal antibody or its fragment or a derivative thereof, which causes an antigen-antibody reaction with an epitope containing one or more amino acid residues selected from the 2nd to 14th amino acid residues from the N-terminal of the amino acid sequence of Neisseria gonorrhoeae L7/L12 shown in SEQ ID NO:1,
wherein the other of the capture antibody and the detection antibody is a second monoclonal antibody or its fragment or a derivative thereof, which causes an antigen-antibody reaction with an epitope containing one or more amino acid residues selected from the 102nd to 123rd amino acid residues from the N-terminal of the amino acid sequence of Neisseria gonorrhoeae L7/L12 shown in SEQ ID NO:1.

2. The kit according to claim 1, wherein the second monoclonal antibody comprises:
as the amino acid sequence of a heavy chain variable region, an amino acid sequence having a homology of 80% or more to the amino acid sequence of SEQ ID NO:7, and,
as the amino acid sequence of a light chain variable region, an amino acid sequence having a homology of 80% or more to the amino acid sequence of SEQ ID NO:8.

3. The kit according to claim 1 or 2, wherein the first monoclonal antibody comprises:
as the amino acid sequence of a heavy chain variable region, an amino acid sequence having a homology of 80% or more to the amino acid sequence of SEQ ID NO:9, and
as the amino acid sequence of a light chain variable region, an amino acid sequence having a homology of 80% or more to the amino acid sequence of SEQ ID NO:10; or
as the amino acid sequence of a heavy chain variable region, an amino acid sequence having a homology of 80% or more to the amino acid sequence of SEQ ID NO:11, and
as the amino acid sequence of a light chain variable region, an amino acid sequence having a homology of 80% or more to the amino acid sequence of SEQ ID NO:12.

4. The kit according to any one of claims 1 to 3, wherein the capture antibody is the first monoclonal antibody and the detection antibody is the second monoclonal antibody.

5. The kit according to any one of claims 1 to 3, wherein the detection antibody is the first monoclonal antibody and the capture antibody is the second monoclonal antibody.

6. A kit for detecting Neisseria gonorrhoeae in a sample, comprising:
a capture antibody for capturing a Neisseria gonorrhoeae-derived antigen in the sample; and
detection antibody having a detection label for labeling the Neisseria gonorrhoeae-derived antigen in the sample,
wherein Neisseria gonorrhoeae is detected via an immune reaction between the capture antibody, and the detection antibody to form a structure sandwiching the Neisseria gonorrhoeae-derived antigen,
wherein the Neisseria gonorrhoeae-derived antigen is a ribosomal protein L7/L12 of Neisseria gonorrhoeae, and
wherein the capture antibody and the detection antibody are selected from:
(1) a combination where the capture antibody is (a) or (b) below, and the labeling antibody is (c) below; or
(2) a combination where the labeling antibody is (a) or (b) below, and the capture antibody is (c) below.
(a) An antibody comprising:
as the amino acid sequence of a heavy chain variable region, an amino acid sequence having a homology of 80% or more to the amino acid sequence of SEQ ID NO:11, and,
as the amino acid sequence of a light chain variable region, an amino acid sequence having a homology of 80% or more to the amino acid sequence of SEQ ID NO:12.
(b) An antibody comprising:
as the amino acid sequence of a heavy chain variable region, an amino acid sequence having a homology of 80% or more to the amino acid sequence of SEQ ID NO:9, and,
as the amino acid sequence of a light chain variable region, an amino acid sequence having a homology of 80% or more to the amino acid sequence of SEQ ID NO:10.
(c) An antibody comprising:
as the amino acid sequence of a heavy chain variable region, an amino acid sequence having a homology of 80% or more to the amino acid sequence of SEQ ID NO:7, and,
as the amino acid sequence of a light chain variable region, an amino acid sequence having a homology of 80% or more to the amino acid sequence of SEQ ID NO:8.

7. The kit according to any one of claims 1 to 6, wherein an efficiency in detecting the Neisseria gonorrhoeae-derived antigen more than 10 times higher than the efficiency in detecting a Neisseria meningitidis -derived antigen.

8. The kit according to any one of claims 1 to 6, which does not cause a cross-reaction with bacteria of the genus Mycoplasma, the genus Escherichia, the genus Chlamydia, the genus Pseudomonas, the genus Staphylococcus, the genus Legionella, the genus Streptococcus in the sample.

9. The kit according to any one of claims 1 to 8, wherein the sample is derived from oral fluid or urine.

10. The kit according to claim 9, wherein the sample is derived from urine of a mammal.

11. The kit according to any one of claims 1 to 10, further comprising a carrier for developing the sample and contacting the sample with the detection antibody,
wherein the carrier has a detection region to which the capture antibody is immobilized.

12. The kit according to claim 11, wherein the kit is an immunochromatography kit,
wherein the kit further comprises a conjugate pad to which the detection antibody is attached.

13. A method for detecting Neisseria gonorrhoeae in a sample using a kit according to any one of claims 1 to 12, comprising the steps of:
(I) capturing and labeling a Neisseria gonorrhoeae-derived antigen in the sample based on an antigen-antibody reaction between the Neisseria gonorrhoeae-derived antigen in the sample, the capture antibody, and the detection antibody; and
(II) detecting the Neisseria gonorrhoeae-derived antigen in the sample by using the detection label.

14. The method according to claim 13, wherein step (I) comprises the steps of:
(Ia-1) making the sample into contact with the detection antibody to thereby label the Neisseria gonorrhoeae-derived antigen in the sample based on an antigen-antibody reaction between the detection antibody and the Neisseria gonorrhoeae-derived antigen in the sample; and
(Ia-2) making the sample containing the Neisseria gonorrhoeae-derived antigen labeled with the detection antibody into contact with the capture antibody to thereby capture the Neisseria gonorrhoeae-derived antigen in the sample based on an antigen-antibody reaction between the capture antibody and a complex of the Neisseria gonorrhoeae-derived antigen and the detection antibody.

15. The method according to claim 13, wherein step (I) comprises the steps of:
(Ib-1) making the sample into contact with the capture antibody to thereby capture the Neisseria gonorrhoeae-derived antigen in the sample based on an antigen-antibody reaction between the capture antibody and the Neisseria gonorrhoeae-derived antigen in the sample; and
(Ib-2) making the sample containing the Neisseria gonorrhoeae-derived antigen captured by the capture antibody into contact with the detection antibody to thereby label the Neisseria gonorrhoeae-derived antigen in the sample based on an antigen-antibody reaction between the detection antibody and a complex of the Neisseria gonorrhoeae-derived antigen and the capture antibody.

16. The method according to any one of claims 13 to 15, further comprising the step of, before making the detection antibody and/or the capture antibody into contact with the sample, lysing the bacteria in the sample using a lysing agent,
wherein the lysing agent is one or more selected from the group consisting of non-ionic surfactant, zwitterionic surfactant, anionic surfactant, lysozyme, lysostaphin, pepsin, glucosidase, galactosidase, achromopeptidase, and β-N-acetyl glucosaminase.

17. The method according to any one of claims 13 to 16, whose detection limit of Neisseria gonorrhoeae in a sample is 5e4 cfu/mL or less.

18. A method for producing a kit according to claim 11, comprising the step of immobilizing the capture antibody to a detection region of a carrier.

19. A method for producing a kit according to claim 12, comprising the steps of:
immobilizing the capture antibody to a detection region of a carrier;
attaching the detection antibody to a conjugate pad; and
arranging the conjugate pad at a position upstream of the detection region of the carrier.

20. The method according to claim 18 or 19, further comprising the step of adding the detection label to one of the first and second monoclonal antibodies to thereby prepare the detection antibody,
wherein the other of the first and second monoclonal antibodies is immobilized to the detection region of the carrier as the capture antibody.

21. The method according to claim 20, wherein the first monoclonal antibody is the first monoclonal antibody according to claim 1,
wherein the method further comprises the step of obtaining the first monoclonal antibody by inoculating an animal with an epitope polypeptide having an epitope containing one or more amino acid residues selected from the 2nd to 14th amino acid residues from the N-terminal of the amino acid sequence of Neisseria gonorrhoeae L7/L12 shown in SEQ ID NO:1, obtaining an antiserum containing an antibody which causes an antigen-antibody reaction with the epitope polypeptide, and purifying and separating the antibody from the antiserum.

22. The method according to claim 20 or 21, wherein the second monoclonal antibody is the second monoclonal antibody according to claim 1,
wherein the method further comprises the step of obtaining the second monoclonal antibody by inoculating an animal with an epitope polypeptide having an epitope containing one or more amino acid residues selected from the 102nd to 123rd amino acid residues from the N-terminal of the amino acid sequence of Neisseria gonorrhoeae L7/L12 shown in SEQ ID NO:1, obtaining an antiserum containing an antibody which causes an antigen-antibody reaction with the epitope polypeptide, and purifying and separating the antibody from the antiserum.
